# EUROPEAN PATENT APPLICATION

(11) **EP 2 645 326 A1**
(43) Date of publication of application: **02.10.2013**
(21) Application number: 11843700.3
(22) Date of filing: 14.11.2011
(51) Int. Cl.: G06Q 50/24, G06Q 50/22

(54) **INFORMATION PROCESSING DEVICE, METHOD, AND PROGRAM**

(30) Priority: 25.11.2010 JP 2010262153
(71) Applicant: Sony Corporation, Tokyo 108-0075 (JP)
(72) Inventor: FUKUSHI Gakuho, Tokyo 108-0075 (JP); WAKITA Yoshihiro, Tokyo 108-0075 (JP)
(74) Representative: Turner, James Arthur
(86) International application number: PCT/JP2011/076175
(87) International publication number: WO 2012/070418

(57) **Abstract**

[Object] The present invention relates to an information processing apparatus and method, and a program capable of improving convenience in a case where a prescription or a medication notebook is made electronically available.

[Solving Means] An in-hospital device 32 sends information of a prescription to a data server 91 and records the information therein after a patient has been examined. In addition, the in-hospital device 32 sends a dispensing reservation request to the data server 91. The dispensing reservation request includes a pharmacy ID indicating a pharmacy for which a dispensing is requested and a prescription ID. The the data server 91 records dispensing reservation information containing the prescription ID and the pharmacy ID in response to the dispensing reservation request. An in-pharmacy device 62 acquires the dispensing reservation information by monitoring the data server 91 regularly and detects whether a dispensing request has been received. If the dispensing request has been received, the in-pharmacy device 62 acquires the information of the prescription from the data server 91. As a result, dispensing can be started in the pharmacy before the patient comes. The present invention is applicable to an information processing system.

## Description

### Technical Field

The present invention relates to an information processing apparatus and method, and a program, and specifically relates to an information processing apparatus and method, and a program capable of improving convenience in a case where a prescription or a medication notebook is made electronically available.

### Background Art

Although prescriptions issued by doctors and medication notebooks issued by pharmacies are printed on paper media currently, it is believed that those prescriptions and medication notebooks are made electronically available in the near future from the viewpoint of improving convenience and efficiency.

For example, as a technology in relation to medication notebooks, there is proposed a technology of previously recording information such as names of drugs and administration routes, creating drug guides based on the information, and printing out the drug guides (for example, see Patent Document 1). According to this technology, a part of a drug guide includes information for archival purpose in order to record information on a drug in a notebook or the like owned by a patient.

Patent Document 1: Japanese Patent Application Laid-open No. H11-28877

### Summary of Invention

### Problem to be solved by the Invention

However, because prescriptions and medication notebooks simply made electrically available are no more than replacement of paper media, it is impossible to make them more convenient than functions of existing prescriptions and medication notebooks. Because of this, doctors, pharmacists, and patients have the following inconveniences, respectively.

That is, speaking as doctors, because a patient manages dose of a drug, it is difficult to take effective action against improvement of patient's drug compliance. Because a patient manages return visit to the doctor, if the patient interrupts visit to the doctor inappropriately based on self-judgment, treatment effect may be decreased.

Further, speaking as pharmacists, because a pharmacist cannot start to dispense until a patient hands in a prescription, the patient has to wait for a long time, which results in lower customer satisfaction most of the time. Similarly, speaking as patient, a patient is bored of waiting at a pharmacy.

Further, speaking as patients, because a patient manages dose of a drug, the patient may, for example, forget to dose a drug and treatment effect may be thus decreased, or the patient may interrupt visit to the doctor inappropriately based on self-judgment and treatment effect may be thus decreased.

The present invention has been made in view of the above-mentioned circumstances, and it is an object of the present invention to improve convenience when a prescription or a medication notebook is made electronically available.

### Means for solving the Problem

An information processing apparatus according to a first aspect of the present invention includes: a creating means for creating a dispensing reservation request, the dispensing reservation request requesting to reserve dispensing of a drug prescribed based on a prescription issued to a patient, the dispensing reservation request including identification information identifying the patient, prescription information on the prescription, and drug identification information identifying the drug of which reservation is requested; and a sending means for sending the dispensing reservation request.

The information processing apparatus may further include an obtaining means for causing a server managing the prescription information to send the identification information, to thereby obtain the prescription information of the patient from the server.

The drug identification information may be information identifying the type of drug, or information showing acceptance/no acceptance of change to generics.

The obtaining means may further obtain dispensing history information on the drug prescribed to the patient, from the server, the information processing apparatus may further include a display means for displaying a list of pharmacies to which dispensing of drugs was requested by the patient in the past, the dispensing history information including the list, and the dispensing reservation request may further include pharmacy identification information identifying a pharmacy to which dispensing reservation of the drug is requested, the pharmacy being selected from the list of the pharmacies.

An information processing method or a program according to the first aspect of the present invention includes the steps of: creating a dispensing reservation request, the dispensing reservation request requesting to reserve dispensing of a drug prescribed based on a prescription issued to a patient, the dispensing reservation request including identification information identifying the patient, prescription information on the prescription, and drug identification information identifying the drug of which reservation is requested; and sending the dispensing reservation request.

According to the first aspect of the present invention, created is a dispensing reservation request, the dispensing reservation request requesting to reserve dispensing of a drug prescribed based on a prescription issued to a patient, the dispensing reservation request including identification information identifying the patient, prescription information on the prescription, and drug identification information identifying the drug of which reservation is requested; and sent is the dispensing reservation request.

An information processing apparatus according to a second aspect of the present invention includes: a recording means for recording prescription information on a prescription issued to a patient and dispensing history information on a drug prescribed to the patient, in relation with identification information identifying the patient; a sending means for sending, in a case where a request to send the prescription information and the dispensing history information including the identification information is received, the prescription information and the dispensing history information recorded in relation with the identification information, to an apparatus that sent the request, in response to the request; a receiving means for receiving a dispensing reservation request sent from the apparatus, the dispensing reservation request requesting to dispensing reservation of a drug prescribed based on the prescription, the dispensing reservation request including pharmacy identification information identifying a pharmacy to which dispensing reservation of a drug is requested, the pharmacy being selected from pharmacies to which dispensing of drugs was requested by the patient in the past, the pharmacies being in at least the identification information, the prescription information, and the dispensing history information; and a creating means for creating, in response to the dispensing reservation request, prescription identification information identifying the prescription, drug identification information identifying a drug of which dispensing reservation is requested, and dispensing reservation information including the pharmacy identification information, and for causing the recording means to record the dispensing reservation information in relation with the identification information, in which the sending means sends the dispensing reservation information to an apparatus at a pharmacy identified by the pharmacy identification information, and sends the prescription information of a prescription identified by the prescription identification information in the dispensing reservation information, to the apparatus at the pharmacy, in response to a request from the apparatus at the pharmacy.

The drug identification information may be information identifying the type of drug, or information showing acceptance/no acceptance of change to generics.

An information processing method or a program according to the second aspect of the present invention includes the steps of: recording prescription information on a prescription issued to a patient and dispensing history information on a drug prescribed to the patient, in relation with identification information identifying the patient; sending, in a case where a request to send the prescription information and the dispensing history information including the identification information is received, the prescription information and the dispensing history information recorded in relation with the identification information, to an apparatus that sent the request, in response to the request; receiving a dispensing reservation request sent from the apparatus, the dispensing reservation request requesting to dispensing reservation of a drug prescribed based on the prescription, the dispensing reservation request including pharmacy identification information identifying a pharmacy to which dispensing reservation of a drug is requested, the pharmacy being selected from pharmacies to which dispensing of drugs was requested by the patient in the past, the pharmacies being in at least the identification information, the prescription information, and the dispensing history information; creating, in response to the dispensing reservation request, prescription identification information identifying the prescription, drug identification information identifying a drug of which dispensing reservation is requested, and dispensing reservation information including the pharmacy identification information, and causing the recording means to record the dispensing reservation information in relation with the identification information; and sending the dispensing reservation information to an apparatus at a pharmacy identified by the pharmacy identification information, and sending the prescription information of a prescription identified by the prescription identification information in the dispensing reservation information, to the apparatus at the pharmacy, in response to a request from the apparatus at the pharmacy.

According to the second aspect of the present invention, recorded is prescription information on a prescription issued to a patient and dispensing history information on a drug prescribed to the patient, in relation with identification information identifying the patient; sent is, in a case where a request to send the prescription information and the dispensing history information including the identification information is received, the prescription information and the dispensing history information recorded in relation with the identification information, to an apparatus that sent the request, in response to the request; received is a dispensing reservation request sent from the apparatus, the dispensing reservation request requesting to dispensing reservation of a drug prescribed based on the prescription, the dispensing reservation request including pharmacy identification information identifying a pharmacy to which dispensing reservation of a drug is requested, the pharmacy being selected from pharmacies to which dispensing of drugs was requested by the patient in the past, the pharmacies being in at least the identification information, the prescription information, and the dispensing history information; created is, in response to the dispensing reservation request, prescription identification information identifying the prescription, drug identification information identifying a drug of which dispensing reservation is requested, and dispensing reservation information including the pharmacy identification information, and causing the recording means to record the dispensing reservation information in relation with the identification information; and sent is the dispensing reservation information to an apparatus at a pharmacy identified by the pharmacy identification information, and sending the prescription information of a prescription identified by the prescription identification information in the dispensing reservation information, to the apparatus at the pharmacy, in response to a request from the apparatus at the pharmacy.

An information processing apparatus according to a third aspect of the present invention includes: a receiving means for receiving, from a server managing prescription information on a prescription issued to a patient in relation with identification information identifying the patient, dispensing reservation information including prescription identification information identifying the prescription in the prescription information, and including drug identification information identifying a drug of which dispensing reservation is requested; an obtaining means for obtaining, from the server, the prescription information on the prescription identified by the prescription identification information, based on the prescription identification information in the dispensing reservation information; and a sending means for sending information indicating that the drug dispensing reservation is accepted, to the server.

The drug identification information may be information identifying the type of drug, or information showing acceptance/no acceptance of change to generics.

The information processing apparatus may further include a recording means for recording stock information showing stock status of drugs at the predetermined pharmacy, in which the sending means may send, in a case where a stock amount of the drug indicated by the stock information is larger than the amount of the drug prescribed based on the prescription corresponding to the prescription information, information that the drug dispensing reservation is accepted.

An information processing method or a program according to the third aspect of the present invention includes the steps of: receiving, from a server managing prescription information on a prescription issued to a patient in relation with identification information identifying the patient, dispensing reservation information including prescription identification information identifying the prescription in the prescription information, and including drug identification information identifying a drug of which dispensing reservation is requested; obtaining, from the server, the prescription information on the prescription identified by the prescription identification information, based on the prescription identification information in the dispensing reservation information; and sending information indicating that the drug dispensing reservation is accepted, to the server.

According to the third aspect of the present invention, received is, from a server managing prescription information on a prescription issued to a patient in relation with identification information identifying the patient, dispensing reservation information including prescription identification information identifying the prescription in the prescription information, and including drug identification information identifying a drug of which dispensing reservation is requested; obtained is, from the server, the prescription information on the prescription identified by the prescription identification information, based on the prescription identification information in the dispensing reservation information; and sent is information indicating that the drug dispensing reservation is accepted, to the server.

An information processing apparatus according to a fourth aspect of the present invention includes: an obtaining means for obtaining, from a recording means recording examination reservation information on examination reservation including an examination appointment day of a patient at a medical institution in relation with identification information identifying the patient, the examination reservation information; a creating means for creating an examination reservation notice notifying the patient of the examination appointment day based on the examination reservation information, in a case where the examination appointment day in the examination reservation information satisfies a predetermined condition; and a sending means for sending the examination reservation notice to the patient's contact information identified by the identification information in relation with the examination reservation information, as a destination.

The creating means may create email or a voice message, as the examination reservation notice.

The examination reservation information includes a message from the medical institution to the patient, and the creating means may create the examination reservation notice including the message.

An information processing method or a program according to the fourth aspect of the present invention includes the steps of: obtaining, from a recording means recording examination reservation information on examination reservation including an examination appointment day of a patient at a medical institution in relation with identification information identifying the patient, the examination reservation information; creating an examination reservation notice notifying the patient of the examination appointment day based on the examination reservation information, in a case where the examination appointment day in the examination reservation information satisfies a predetermined condition; and sending the examination reservation notice to the patient's contact information identified by the identification information in relation with the examination reservation information, as a destination.

According to the fourth aspect of the present invention, obtained is, from a recording means recording examination reservation information on examination reservation including an examination appointment day of a patient at a medical institution in relation with identification information identifying the patient, the examination reservation information; created is an examination reservation notice notifying the patient of the examination appointment day based on the examination reservation information, in a case where the examination appointment day in the examination reservation information satisfies a predetermined condition; and sent is the examination reservation notice to the patient's contact information identified by the identification information in relation with the examination reservation information, as a destination.

An information processing apparatus according to a fifth aspect of the present invention includes: a receiving means for receiving, from a server managing examination reservation information on examination reservation including an examination appointment day of a patient at a medical institution in relation with identification information identifying the patient, examination reservation notice notifying the patient of the examination appointment day, the examination reservation notice being sent in a case where the examination appointment day in the examination reservation information satisfies a predetermined condition; and an informing means for informing the patient of the examination appointment day based on the received examination reservation notice.

The examination reservation notice may be email, and the informing means may display the email to thereby inform the patient of the examination appointment day.

The examination reservation notice may be a voice message, and the informing means may output the voice message to thereby inform the patient of the examination appointment day.

An information processing method or a program according to the fifth aspect of the present invention includes the steps of: receiving, from a server managing examination reservation information on examination reservation including an examination appointment day of a patient at a medical institution in relation with identification information identifying the patient, examination reservation notice notifying the patient of the examination appointment day, the examination reservation notice being sent in a case where the examination appointment day in the examination reservation information satisfies a predetermined condition; and informing the patient of the examination appointment day based on the received examination reservation notice.

According to the fifth aspect of the present invention, received is, from a server managing examination reservation information on examination reservation including an examination appointment day of a patient at a medical institution in relation with identification information identifying the patient, examination reservation notice notifying the patient of the examination appointment day, the examination reservation notice being sent in a case where the examination appointment day in the examination reservation information satisfies a predetermined condition; and the patient is informed of the examination appointment day based on the received examination reservation notice.

An information processing apparatus according to a sixth aspect of the present invention includes: an obtaining means for obtaining, from a recording means recording dispensing history information on a drug including information on administration timing of the drug prescribed to a patient in relation with identification information identifying the patient, the dispensing history information; a creating means for creating an administration notice urging the patient to administer the drug based on the dispensing history information, in a case where information showing the administration timing in the dispensing history information satisfies a predetermined condition; and a sending means for sending the administration notice to the patient's contact information identified by the identification information in relation with the dispensing history information, as a destination.

The creating means may create email including the amount of the drug to be administered and an image of the drug, as the administration notice.

The creating means may create a voice message urging the patient to administer the drug by a predetermined amount, as the administration notice.

An information processing method or a program according to the sixth aspect of the present invention includes the steps of: obtaining, from a recording means recording dispensing history information on a drug including information on administration timing of the drug prescribed to a patient in relation with identification information identifying the patient, the dispensing history information; creating an administration notice urging the patient to administer the drug based on the dispensing history information, in a case where information showing the administration timing in the dispensing history information satisfies a predetermined condition; and sending the administration notice to the patient's contact information identified by the identification information in relation with the dispensing history information, as a destination.

According to the sixth aspect of the present invention, obtained is, from a recording means recording dispensing history information on a drug including information on administration timing of the drug prescribed to a patient in relation with identification information identifying the patient, the dispensing history information; created is an administration notice urging the patient to administer the drug based on the dispensing history information, in a case where information showing the administration timing in the dispensing history information satisfies a predetermined condition; and sent is the administration notice to the patient's contact information identified by the identification information in relation with the dispensing history information, as a destination.

An information processing apparatus according to a seventh aspect of the present invention includes: a receiving means for receiving, from a server managing dispensing history information on the drug including information showing administration timing of a drug prescribed to a patient in relation with identification information identifying the patient, an administration notice urging the patient to administer the drug, the administration notice being sent in a case where information indicating the administration timing in the dispensing history information satisfies a predetermined condition; and an informing means for informing the patient of administration of the drug based on the received administration notice.

The administration notice may be email including the amount of the drug to be administered and an image of the drug, and the informing means may display the email to thereby inform the patient of administration of the drug.

The administration notice may be a voice message urging the patient to administer the drug by a predetermined amount, and the informing means may output the voice message to thereby inform the patient of administration of the drug.

An information processing method or a program according to the seventh aspect of the present invention includes the steps of: receiving, from a server managing dispensing history information on the drug including information showing administration timing of a drug prescribed to a patient in relation with identification information identifying the patient, an administration notice urging the patient to administer the drug, the administration notice being sent in a case where information indicating the administration timing in the dispensing history information satisfies a predetermined condition; and informing the patient of administration of the drug based on the received administration notice.

According to the seventh aspect of the present invention, received is, from a server managing dispensing history information on the drug including information showing administration timing of a drug prescribed to a patient in relation with identification information identifying the patient, an administration notice urging the patient to administer the drug, the administration notice being sent in a case where information indicating the administration timing in the dispensing history information satisfies a predetermined condition; and the patient is informed of administration of the drug based on the received administration notice.

### Effect of the Invention

According to the first aspect to the seventh aspect of the present invention, it is possible to improve convenience in a case where a prescription or a medication notebook is made electronically available.

### Brief Description of Drawings

[Fig. 1] A diagram showing a configuration example of an information processing system according to an embodiment of the present invention.
[Fig. 2] A diagram showing a configuration example of a mobile terminal device.
[Fig. 3] A diagram showing a configuration example of an in-hospital database.
[Fig. 4] A diagram showing an example of a patient basic information table.
[Fig. 5] A diagram showing an example of a prescription issued history information table.
[Fig. 6] A diagram showing an example of a drug prescribing history information table.
[Fig. 7] A diagram showing a configuration example of an in-pharmacy database.
[Fig. 8] A diagram showing an example of a drug dispensing history information table.
[Fig. 9] A diagram showing an example of a drug stock information table.
[Fig. 10] A diagram showing a configuration example of a prescription/dispensing database.
[Fig. 11] A diagram showing an example of an identification information/patient correspondence database.
[Fig. 12] A diagram showing an example of a patient basic information database.
[Fig. 13] A diagram showing an example of a doctor prescription information database.
[Fig. 14] A diagram showing an example of a pharmacist dispensing information database.
[Fig. 15] A diagram showing an example of a return-visit reservation information database.
[Fig. 16] A diagram showing an example of a dispensing reservation information table.
[Fig. 17] A flowchart for explaining dispensing reservation process and dispensing reservation writing process.
[Fig. 18] A flowchart for explaining dispensing acceptance process.
[Fig. 19] A flowchart for explaining dispensing reservation information sending process.
[Fig. 20] A flowchart for explaining return-visit reservation requesting process and return-visit reservation information recording process.
[Fig. 21] A flowchart for explaining return-visit notifying process.
[Fig. 22] A flowchart for explaining return-visit notice receiving process.
[Fig. 23] A flowchart for explaining administration notifying process.
[Fig. 24] A flowchart for explaining administration notice receiving process.
[Fig. 25] A diagram showing a configuration example of a computer. Mode for Carrying Out the Invention

Hereinafter, an embodiment of the present invention will be described with reference to the drawings.

### <Configuration example of information processing system>

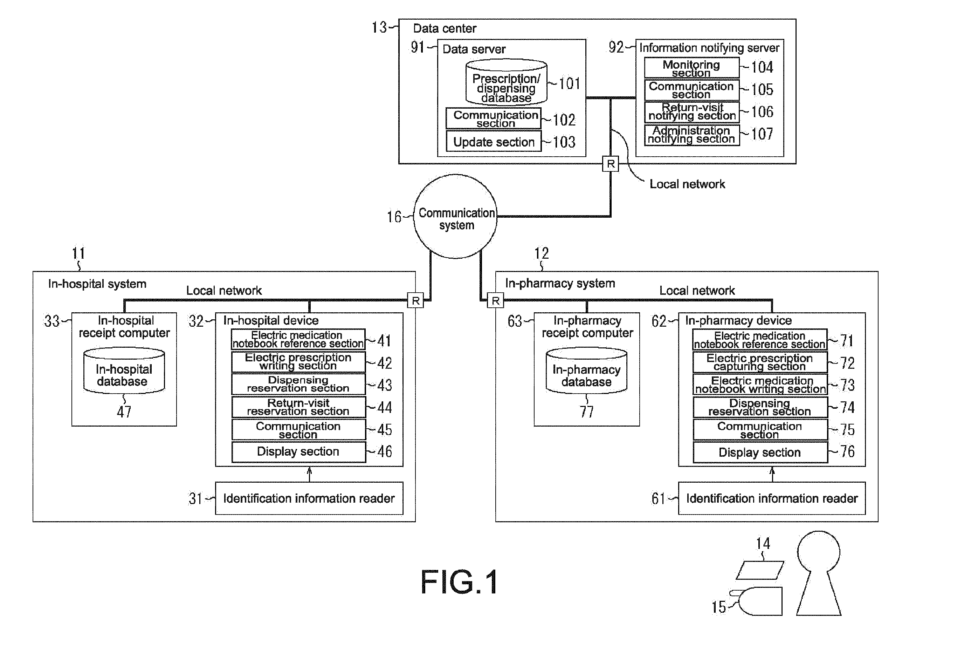

Fig. 1 is a diagram showing the configuration example of an information processing system according to an embodiment of the present invention.

An information processing system includes an in-hospital system 11, an in-pharmacy system 12, a data center 13, and an IC (Integrated Circuit) card 14 and a mobile terminal device 15 owned by a user i.e., a patient. Further, the in-hospital system 11 to the data center 13 are connected each other via a communication system 16 including a wired/wireless network such as the Internet.

The in-hospital system 11 is provided in a hospital, which the patient who owns the IC card 14 and the mobile terminal device 15 visits, and includes an identification information reader 31, an in-hospital device 32, and an in-hospital receipt computer 33. Further, the in-hospital device 32 and the in-hospital receipt computer 33 are connected each other via a local network, and the local network is connected to the communication system 16 via a router and the like.

The identification information reader 31 wirelessly communicates with the IC card 14 of the patient, retrieves identification information, which is an ID unique to the patient, from the IC card 14, and supplies the identification information to the in-hospital device 32.

Note that a medium, from which identification information identifying a patient is obtained, may not be an IC card in which identification information is recorded directly, but may be a non-rewritable and non-replaceable unique medium. For example, in a case of obtaining unique identification information from biological information, a medium, from which identification information is obtained, is a part or all of a human body or the like.

The in-hospital device 32 includes, for example, a computer and the like, and executes various processes based on identification information supplied from the identification information reader 31 and based on operations input by a doctor or the like. The in-hospital device 32 includes an electric medication notebook reference section 41, an electric prescription writing section 42, a dispensing reservation section 43, a return-visit reservation section 44, a communication section 45, and a display section 46.

The electric medication notebook reference section 41 retrieves information on a prescription, information on a drug to be dosed, information on a patient himself, and the like from the data center 13 based on identification information supplied from the identification information reader 31 as a key.

The electric prescription writing section 42 obtains information on a prescription of a patient from the in-hospital receipt computer 33, and writes the obtained information in the data center 13. The dispensing reservation section 43 writes information on dispensing reservation of a drug prescribed to a patient in the data center 13 based on identification information supplied from the identification information reader 31.

The return-visit reservation section 44 executes processes about return-visit reservation of a patient based on identification information supplied from the identification information reader 31. The communication section 45 sends/receives information to/from other devices via a local network or the communication system 16. The display section 46 displays various information and images.

The in-hospital receipt computer 33 includes an in-hospital database 47, and updates information recorded in the in-hospital database 47 based on operations input by a doctor or the like. For example, information on patients, information on prescriptions, information on drugs prescribed to patients, and the like are recorded in the in-hospital database 47.

Note that the in-hospital receipt computer 33 and the in-hospital database 47 may be physically different apparatuses. Alternatively, the in-hospital receipt computer 33, the in-hospital device 32, and the identification information reader 31 may be in a single apparatus.

The in-pharmacy system 12 is provided in a pharmacy, at which a patient purchases a prescribed drug, and includes an identification information reader 61, an in-pharmacy device 62, and an in-pharmacy receipt computer 63. Further, the in-pharmacy device 62 and the in-pharmacy receipt computer 63 are connected each other via a local network, and the local network is connected to the communication system 16 via a router and the like.

The identification information reader 61 wirelessly communicates with the IC card 14 of a patient, retrieves identification information unique to a patient from the IC card 14, and supplies the identification information to the in-pharmacy device 62.

The in-pharmacy device 62 is, for example, a computer or the like, and executes various processes based on identification information supplied from the identification information reader 61 and based on operations input by a pharmacist or the like. The in-pharmacy device 62 includes an electric medication notebook reference section 71, an electric prescription capturing section 72, an electric medication notebook writing section 73, a dispensing reservation section 74, a communication section 75, and a display section 76.

The electric medication notebook reference section 71 retrieves information on a prescription, information on a drug to be dosed, information on a patient himself, and the like from the data center 13 based on identification information supplied from the identification information reader 61 as a key.

The electric prescription capturing section 72 obtains information on a prescription, information on a patient himself, and the like from the data center 13 based on identification information supplied from the identification information reader 61 as a key, extracts necessary information from the obtained information, and supplies the necessary information to the in-pharmacy receipt computer 63.

The electric medication notebook writing section 73 writes information on a drug dispensed to a patient in the data center 13. The dispensing reservation section 74 captures information on dispensing reservation of a drug prescribed to a patient from the data center 13. The communication section 75 sends/receives information to/from other devices via a local network or the communication system 16. The display section 76 displays various information and images.

The in-pharmacy receipt computer 63 includes an in-pharmacy database 77, and updates information recorded in the in-pharmacy database 77 based on operations input by a pharmacist or the like. For example, information on patients, information on drugs dispensed to patient, information on stock of drugs, and the like are recorded in the in-pharmacy database 77.

Note that the in-pharmacy receipt computer 63 and the in-pharmacy database 77 may be physically different apparatuses. Alternatively, the in-pharmacy receipt computer 63, the in-pharmacy device 62, and the identification information reader 61 may be in a single apparatus.

The data center 13 includes a data server 91 and an information notifying server 92. The data server 91 and the information notifying server 92 are connected each other via a local network, and the local network is connected to the communication system 16 via a router and the like.

The data server 91 includes a prescription/dispensing database 101, a communication section 102, and an update section 103, and various information supplied from the in-hospital system 11 or the in-pharmacy system 12 is recorded in the prescription/dispensing database 101.

The communication section 102 sends/receives information to/from other devices via a local network or the communication system 16. Further, the update section 103 updates various information recorded in the prescription/dispensing database 101.

The information notifying server 92 includes a monitoring section 104, a communication section 105, a return-visit notifying section 106, and an administration notifying section 107.

The monitoring section 104 regularly monitors the prescription/dispensing database 101 of the data server 91 as necessary. The communication section 105 sends/receives information to/from other devices via a local network or the communication system 16.

The return-visit notifying section 106 executes processes about a notice of return-visit reservation to a hospital (examination reservation) for a patient based on a monitoring result monitored by the monitoring section 104. The administration notifying section 107 executes processes about a notice of administration for a patient based on a monitoring result monitored by the monitoring section 104.

Further, in a case of executing processes about a notice of return-visit reservation for a patient or administration notice, the information notifying server 92 communicates with the mobile terminal device 15 of the patient via the communication system 16, and provides various notices. Note that a not-shown base station is connected to the communication system 16, and the base station wirelessly communicates with the mobile terminal device 15 to thereby send information from the information notifying server 92 to the mobile terminal device 15.

Note that a part of processes executed by the in-hospital system 11 to the data center 13 may be realized by manual operations.

### < Configuration example of mobile terminal device>

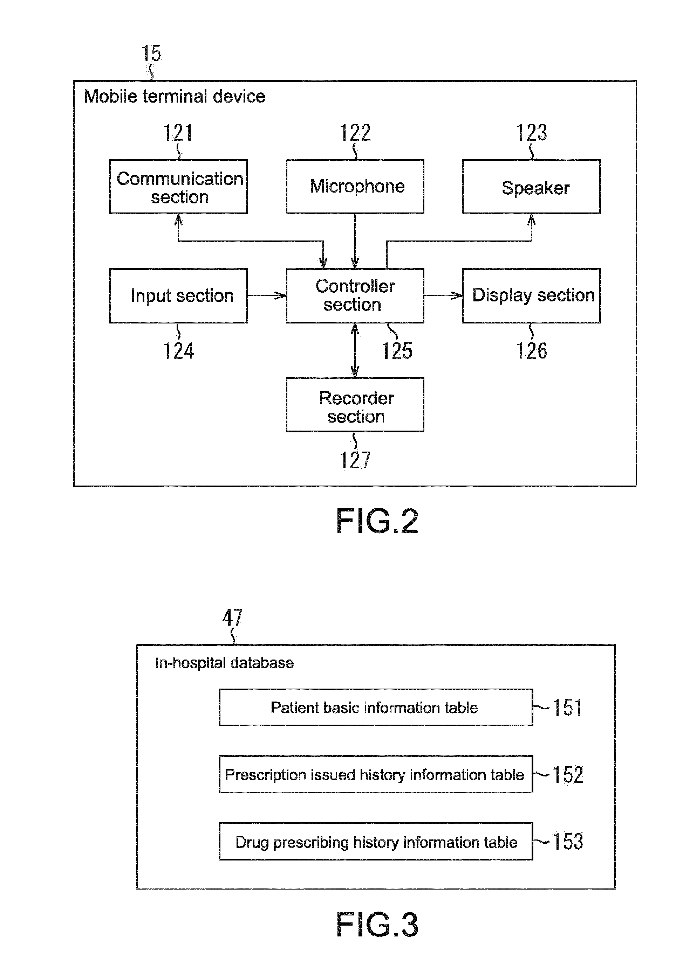

Further, the mobile terminal device 15 of Fig. 1 is configured as shown in Fig. 2, for example.

That is, the mobile terminal device 15 includes a communication section 121, a microphone 122, a speaker 123, an input section 124, a controller section 125, a display section 126, and a recorder section 127.

For example, the mobile terminal device 15 is a mobile phone or the like, and executes telephone-call processes and email sending/receiving processes via a not-shown base station connected to the communication system 16.

The communication section 121 wirelessly communicates with a base station, and sends/receives various information. The microphone 122 collects voice of a patient, and supplies sound data thus obtained to the communication section 121 via the controller section 125. The speaker 123 obtains sound data, which is sent from a base station and received by the communication section 121, from the communication section 121 via the controller section 125, and outputs sound based on the obtained sound data.

The input section 124 includes buttons and the like operated by a patient, and supplies a signal to the controller section 125 based on an operation by a patient. The controller section 125 controls overall behaviors of the mobile terminal device 15. For example, the controller section 125 causes the display section 126 to display email supplied from the communication section 121 and the like, supplies various data supplied from the communication section 121 to the recorder section 127, and causes the recorder section 127 to record the various data.

The display section 126 includes a liquid crystal panel and the like, and displays an image supplied from the controller section 125 and the like. The recorder section 127 includes a nonvolatile memory and the like, and records data supplied from the controller section 125.

### <About in-hospital database>

### [Configuration example of in-hospital database]

Next, specific examples of information recorded in the in-hospital database 47 of the in-hospital receipt computer 33, the in-pharmacy database 77 of the in-pharmacy receipt computer 63, and the prescription/dispensing database 101 of the data server 91 of Fig. 1, respectively, will be described.

First, the in-hospital database 47 of the in-hospital receipt computer 33 will be described. As shown in Fig. 3 for example, a patient basic information table 151, a prescription issued history information table 152, and a drug prescribing history information table 153 are recorded in the in-hospital database 47.

The patient basic information table 151 includes patient basic information, which is information on a patient himself such as the name of a patient. The patient basic information is previously input by operating the in-hospital receipt computer 33 by a doctor or the like.

The prescription issued history information table 152 includes prescription issued history information, which is information on a prescription issued to a patient by a doctor. The drug prescribing history information table 153 includes drug prescribing history information, which is information on a drug prescribed to a patient by a doctor. The prescription issued history information and the drug prescribing history information are input by operating the in-hospital receipt computer 33 by a doctor or the like every time a doctor issues a prescription to a patient.

### [About patient basic information table]

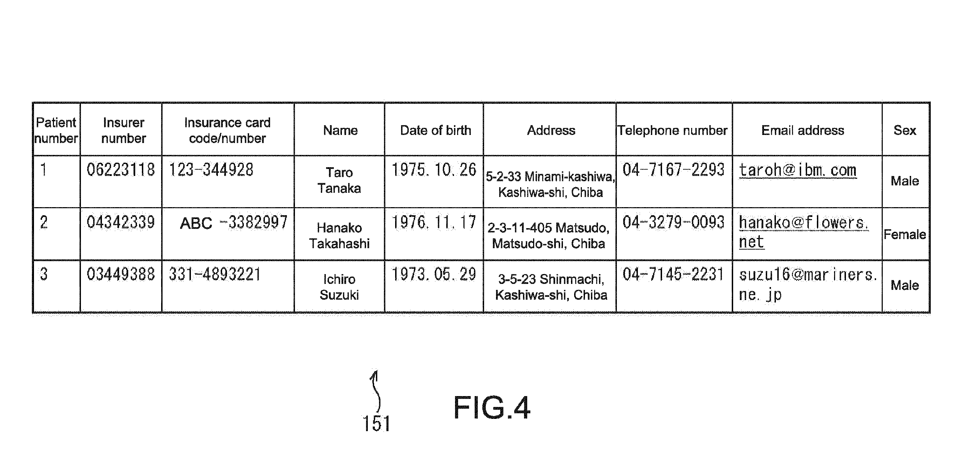

More specifically, as shown in Fig. 4, the patient basic information table 151 includes patient basic information of patients. The patient basic information of patients includes "patient number", "insurer number", "insurance card code/number", "name", "date of birth", "address", "telephone number", "email address", and "sex". Here, "patient number" in the patient basic information is an identification number, which a hospital issues to a patient uniquely.

For example, the patient basic information of a patient whose "patient number" is "1" includes patient number "1", insurer number "06223118", insurance card code/number" 123-344928", name "Taro Tanaka", date of birth "1975/10/26", address "5-2-33 Minami-kashiwa, Kashiwa-shi, Chiba", telephone number "04-7167-2293", email address "taroh@ibm.com", and sex "male".

### [About prescription issued history information table]

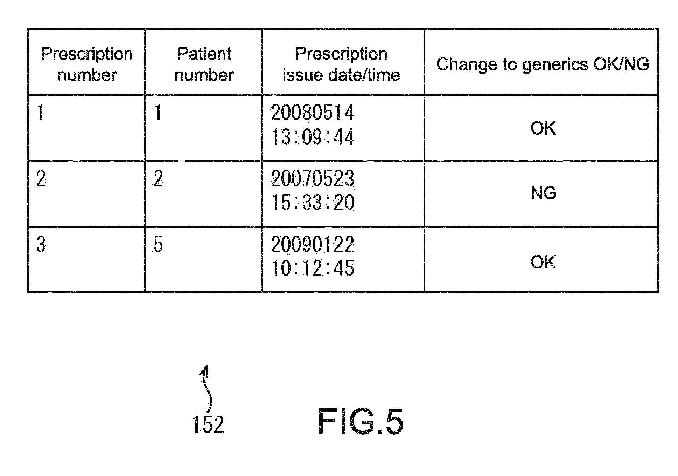

Further, as shown in Fig. 5, the prescription issued history information table 152 of Fig. 3 includes prescription issued history information on a prescription issued to a patient. The prescription issued history information is information held for each prescription in a case where a doctor dispenses a drug as medical practice and issues a prescription.

The prescription issued history information includes "prescription number", "patient number", "prescription issue date/time", and "change to generics OK/NG".

Here, "prescription number" is an identification number, which a hospital uniquely provides to each prescription issued by a doctor. Further, "change to generics OK/NG" is information showing if a doctor allows generics, i.e., generic drugs, or not as an alternative drug for a prescribed drug.

For example, the prescription issued history information of the prescription having "prescription number" of "1" includes prescription number "1", patient number "1", prescription issue date/time "20080514 13:09:44", and change to generics OK/NG "OK".

Therefore, it is understood that the prescription identified by prescription number "1" is issued to the patient, whose patient number of Fig. 4 is "1" and whose name is "Taro Tanaka", on 14 May 2008, 13:09. Further, it is understood that a doctor allows changing a prescribed drug for generics when issuing the prescription.

### [About drug prescribing history information table]

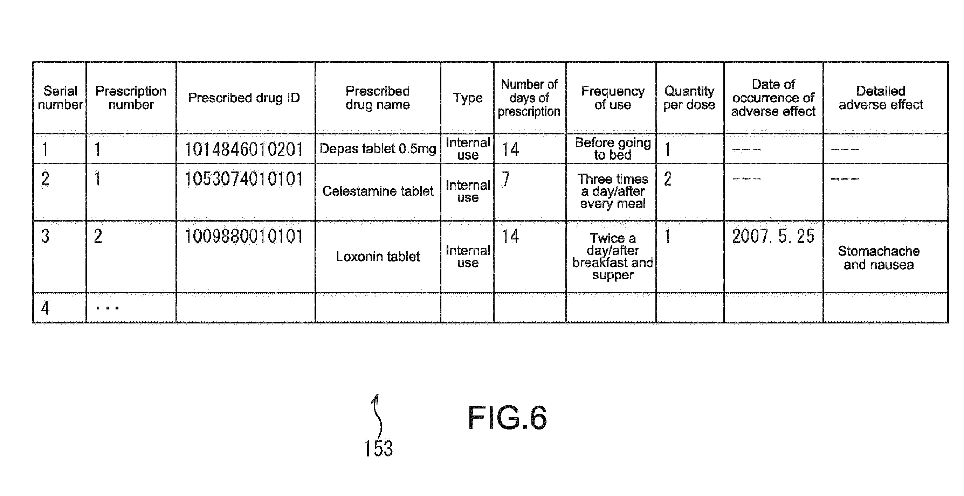

Further, as shown in Fig. 6, the drug prescribing history information table 153 of Fig. 3 includes drug prescribing history information on drugs prescribed to patients by doctors. The drug prescribing history information includes information on an actual drug in a prescription, and information on adverse effects reported by a patient.

That is, each drug prescribing history information includes "serial number", "prescription number", "prescribed drug ID", "prescribed drug name", "type", "number of days of prescription", "frequency of use", "quantity per dose", "date of occurrence of adverse effect", and "detailed adverse effect".

Here, "prescription number" is a number identifying a prescription issued by a doctor, and "prescription number" is a number common to "prescription number" in the prescription issued history information of Fig. 5. Therefore, the drug prescribing history information and the prescription issued history information, which include the same prescription number, include information on the same prescription.

"Prescribed drug ID" is information identifying a prescribed drug, and is, for example, an ID used for a public database such as Standard Drug Master (Hyoujun Iyakuhin Master), or the like.

Note that, with reference to "prescribed drug ID", not only the in-hospital system 11 but also the in-pharmacy system 12 or the data center 13 is capable of identifying a drug based on the prescribed drug ID.

"Type" in the drug prescribing history information is information showing an administration route of a prescribed drug such as internal use or external use, and the like. Note that "type" may include, in addition to an administration route, information showing a product form of a prescribed drug such as tablet or granule.

"Number of days of prescription" in the drug prescribing history information is information showing the number of days of a prescribed drug, and "frequency of use" is information showing timing of dose of a prescribed drugs such as before going to bed or after foods, and the number of administration per day. Further, "quantity per dose" in the drug prescribing history information is information showing administration quantity of a prescribed drug per dose such as one tablet or one packet.

Further, "date of occurrence of adverse effect" and "detailed adverse effect" are date/time of occurrence of an adverse effect due to dose of a prescribed drug by a patient, and information showing the adverse effect in detail such as, for example, "stomachache and nausea", respectively. Note that, when a doctor, a nurse, or the like receives a report of an adverse effect from a patient, he writes notes in the fields of "date of occurrence of adverse effect" and "detailed adverse effect".

Here, the adverse effect reported by a patient is a symptom due to a prescribed drug. A doctor or the like receives a report of the symptom when he examines the patient at a later date. "Date of occurrence of adverse effect" and "detailed adverse effect" are written by operating the in-hospital receipt computer 33 by a doctor or the like.

Note that the drug prescribing history information in the drug prescribing history information table 153 is created for each prescribed drug. That is, in a case where a plurality of drugs are prescribed based on the same prescription, the drug prescribing history information is created for each drug (prescribed drug).

For example, the drug prescribing history information of serial number "1" and the drug prescribing history information of serial number "2" include the same prescription number "1". Therefore, it is understood that the prescribed drug "Depas tablet 0.5mg" in the drug prescribing history information of serial number "1" is a drug prescribed based on a prescription same as that of prescribed drug "Celestamine tablet" in the drug prescribing history information of serial number "2".

### <About in-pharmacy database>

### [Configuration example of in-pharmacy database]

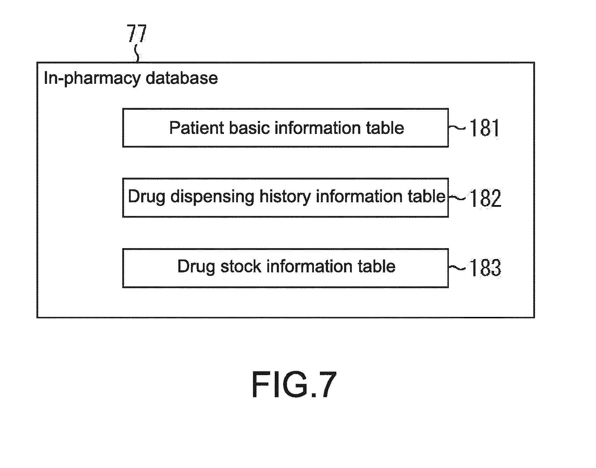

Next, the in-pharmacy database 77 of the in-pharmacy receipt computer 63 will be described. For example, as shown in Fig. 7, a patient basic information table 181, a drug dispensing history information table 182, and a drug stock information table 183 are recorded in the in-pharmacy database 77.

The patient basic information table 181 includes patient basic information, which is information on a patient himself such as the name of a patient. The patient basic information is previously input by operating the in-pharmacy receipt computer 63 by a pharmacist or the like.

For example, the patient basic information table 181 is information similar to the patient basic information table 151 of Fig. 4. Note that the patient basic information table 181 and the patient basic information table 151 may be created individually by each hospital or pharmacy, or the same table may be used for those tables.

The drug dispensing history information table 182 includes drug dispensing history information, which is information on a drug dispensed to a patient by a pharmacist. The drug dispensing history information is input by operating the in-pharmacy receipt computer 63 by a pharmacist or the like every time a pharmacist dispenses a drug based on a prescription.

The drug stock information table 183 is information of the stock status of drugs in a pharmacy, in which the in-pharmacy system 12 is provided, and the drug stock information table 183 includes drug stock information showing the stock status of drugs for each drug. The drug stock information table 183 is updated every time a drug is dispensed.

### [About drug dispensing history information table]

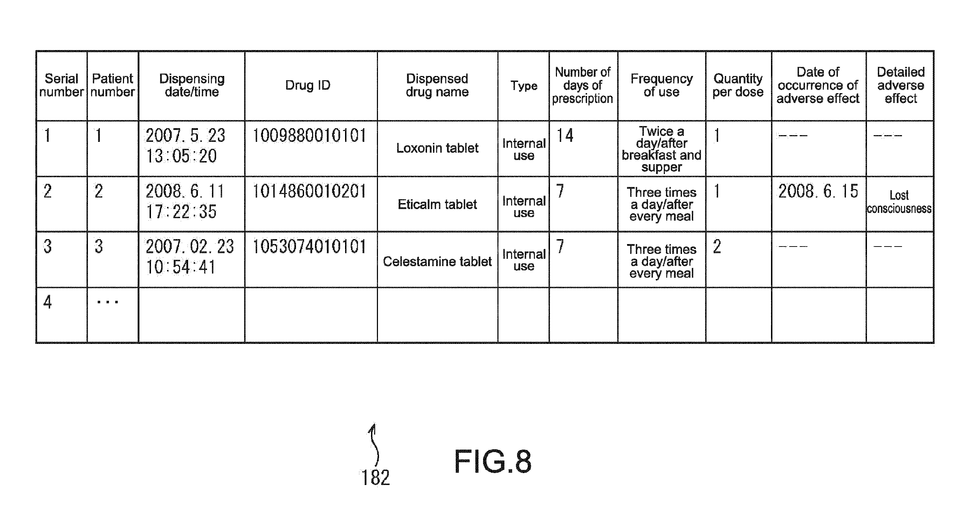

As shown in Fig. 8, the drug dispensing history information table 182 of Fig. 7 includes drug dispensing history information of a drug dispensed by a pharmacist. Each drug dispensing history information includes "serial number", "patient number", "dispensing date/time", "dispensed drug ID", "dispensed drug name", "type", "number of days of prescription", "frequency of use", "quantity per dose", "date of occurrence of adverse effect", and "detailed adverse effect".

Here, "patient number" is the same as the patient number in the patient basic information of the patient basic information table 181. Because of this, by searching for patient basic information in relation to the patient number same as "patient number" in the drug dispensing history information, it is possible to specify to which patient the drug in the drug dispensing history information is dispensed.

"Dispensed drug ID" is information specifying a dispensed drug, and is, for example, an ID used for a public database such as Standard Drug Master (Hyoujun Iyakuhin Master), or the like. Note that the drug identified by "dispensed drug ID" is a unique name of a drug such as a particular product.

Further, "type", "number of days of prescription", "frequency of use", "quantity per dose", "date of occurrence of adverse effect", and "detailed adverse effect" in the drug dispensing history information are information similar to "type" to "detailed adverse effect" in the drug prescribing history information of Fig. 6.

Note that, when a pharmacist or the like receives a report of an adverse effect from a patient, he writes notes in the fields of "date of occurrence of adverse effect" and "detailed adverse effect". A pharmacist or the like operates the in-pharmacy receipt computer 63 to thereby write "date of occurrence of adverse effect" and "detailed adverse effect".

Because information on an adverse effect in the drug dispensing history information is written when a pharmacist or the like receives a report, the drug dispensing history information and the drug prescribing history information table 153 in the in-hospital database 47 do not always include information on an adverse effect that the same patient suffers. Note that the data center 13 may allow the in-hospital system 11 and the in-pharmacy system 12 to share information on an adverse effect that each patient suffers.

### [About drug stock information table]

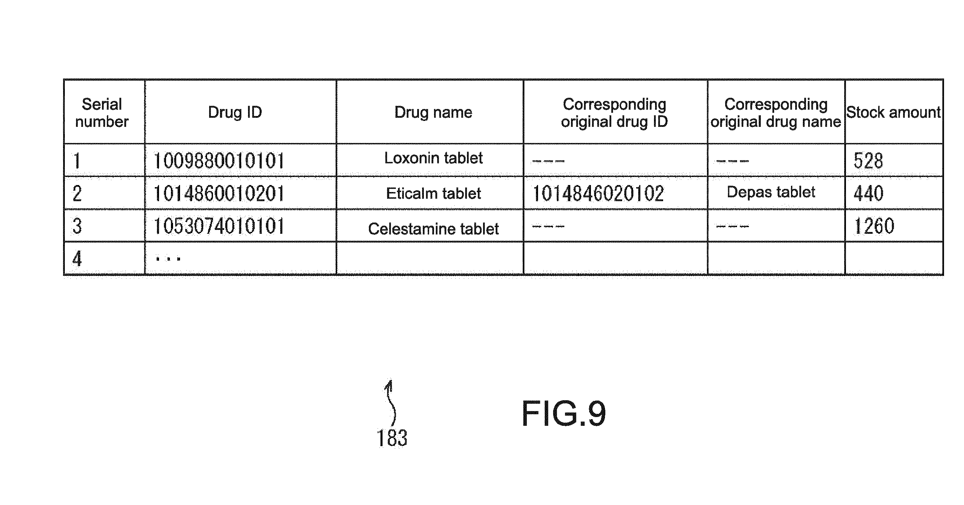

Further, for example, as shown in Fig. 9, the drug stock information table 183 in the in-pharmacy database 77 of Fig. 7 includes drug stock information showing the stock status of each drug (remaining amount of drug) in a pharmacy. The drug stock information of each drug includes "serial number", "drug ID", "drug name", "corresponding original drug ID", "corresponding original drug name", and "stock amount".

Here, "drug ID" is information identifying a drug in a pharmacy, and is, for example, an ID used for a public database such as Standard Drug Master (Hyoujun Iyakuhin Master), or the like. Further, "corresponding original drug ID" is an ID identifying an original drug of a drug identified by "drug ID", and "corresponding original drug name" is the drug name of an original drug identified by "corresponding original drug ID".

Note that "corresponding original drug ID" is written only in a case where the drug identified by "drug ID" is a generic and where an original drug of the drug exists.

### <About prescription/dispensing database>

### [Configuration example of prescription/dispensing database]

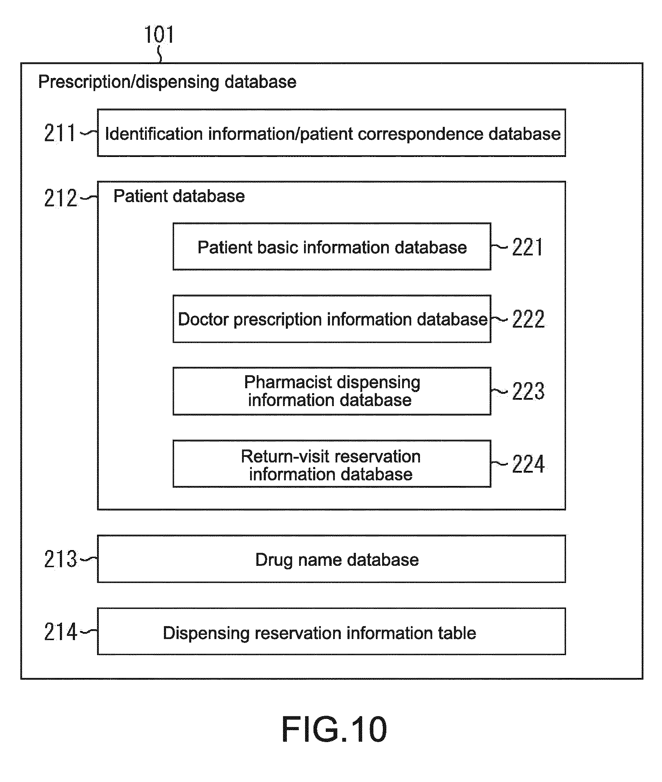

Further, the prescription/dispensing database 101 of the data server 91 of Fig. 1 will be described. For example, as shown in Fig. 10, an identification information/patient correspondence database 211, a patient database 212, a drug name database 213, and a dispensing reservation information table 214 are recorded in the prescription/dispensing database 101.

Identification information recorded in the IC card 14 owned by a patient and information identifying a patient are recorded in relation to each other in the identification information/patient correspondence database 211.

The patient database 212 includes various kinds of information on a patient. Specifically, the patient database 212 includes a patient basic information database 221, a doctor prescription information database 222, a pharmacist dispensing information database 223, and a return-visit reservation information database 224.

Note that, more specifically, each piece of information in the patient database 212 is coded by means of a predetermined method.

The patient basic information database 221 includes information on a patient himself such as the insurer number of a patient, date of birth, and contact information, and the doctor prescription information database 222 includes information on a prescription issued to a patient. Further, the pharmacist dispensing information database 223 includes information on a drug dispensed to a patient, and the return-visit reservation information database 224 includes information on return-visit to a hospital by a patient.

Note that the patient database 212 is recorded for each patient in the identification information/patient correspondence database 211. That is, a plurality of patient databases 212 are recorded in the prescription/dispensing database 101.

The drug name database 213 is a public database such as, for example, Standard Drug Master (Hyoujun Iyakuhin Master). The drug name database 213 includes information such as a drug ID identifying each drug, a drug name, a drug ID of a corresponding generic, a drug name of the generic, unit price of a drug, and a unit of quantity of a drug. The dispensing reservation information table 214 includes information on dispensing reservation of a drug prescribed by a patient.

Note that the identification information/patient correspondence database 211 to the drug name database 213 and the dispensing reservation information table 214 may be recorded in different servers, respectively.

### [About identification information/patient correspondence database]

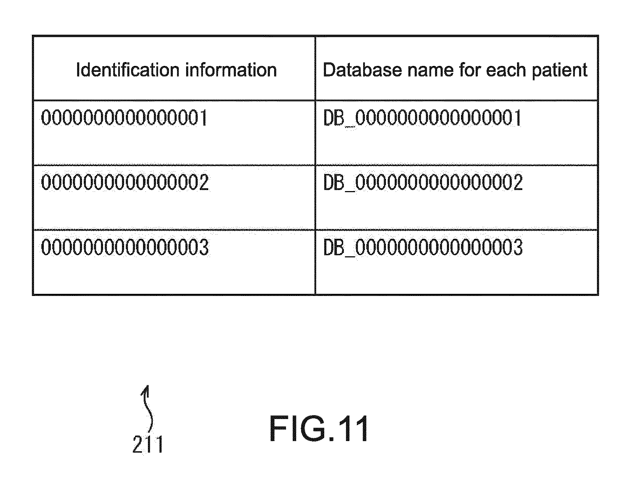

Further, the identification information/patient correspondence database 211 of Fig. 10 includes a correspondence table shown in Fig. 11, for example.

"Identification information" recorded in the IC card 14 owned by a patient and "database name for each patient" are recorded in relation to each other in a correspondence table of Fig. 11. Here, "database name for each patient" is information showing the name of the patient database 212 of a patient identified by "identification information".

As described above, the identification information/patient correspondence database 211 in the data server 91 manages identification information identifying a patient and information on each patient in relation to each other.

Note that, in a case where the name of the patient database 212 for each patient is the same as the name of identification information recorded in the IC card 14 of a patient, it is not necessary to provide the identification information/patient correspondence database 211. Further, the correspondence relation of identification information and the patient database 212 may be secured by means of an appropriate method such that it is impossible to guess the correspondence relation from the outside.

### [About patient basic information database]

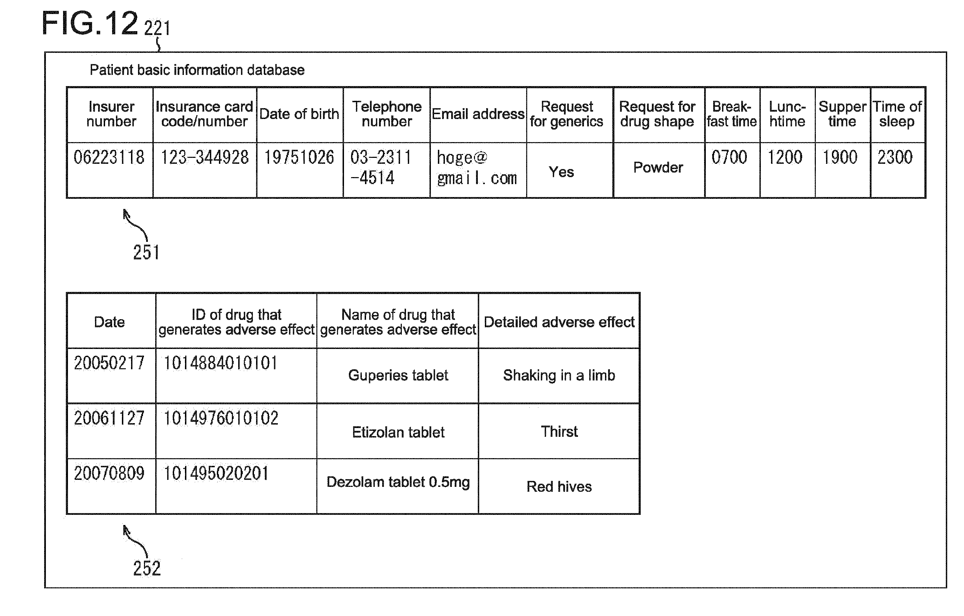

Further, for example, as shown in Fig. 12, the patient basic information database 221 of Fig. 10 includes a patient basic information table 251 and an adverse effect information table 252.

The patient basic information table 251 includes "insurer number", "insurance card code/number", "date of birth", "telephone number", "email address", "request for generics", "request for drug shape", "breakfast time", "lunchtime", "supper time", and "time of sleep" of a patient.

Here, "request for generics" is information showing if a patient requests for a generic, i.e., a generic drug, as an alternative drug for a prescribed drug or not. "Request for drug shape" is information showing the product form (shape) of a prescribed drug that a patient requests, such as powder, tablet, or liquid.

Further, "breakfast time", "lunchtime", "supper time", and "time of sleep" are information showing breakfast time, lunchtime, supper time, and time of sleep of a patient, respectively.

Information in the patient basic information table 251 is previously input by each patient or the like. Note that the data server 91 may create the patient basic information table 251 based on patient basic information obtained from the in-hospital system 11 or the in-pharmacy system 12.

Further, the adverse effect information table 252 includes "date" showing the day of occurrence of an adverse effect, "ID of drug that generates adverse effect" identifying a drug that generates an adverse effect, "name of drug that generates adverse effect" showing the name of a drug that generates an adverse effect, and "detailed adverse effect" showing a specific adverse effect in detail.

The in-hospital system 11 or the in-pharmacy system 12 supplies, as necessary, drug prescribing history information or drug dispensing history information each including information on an adverse effect that a patient suffers, and then the adverse effect information table 252 is updated.

### [About doctor prescription information database]

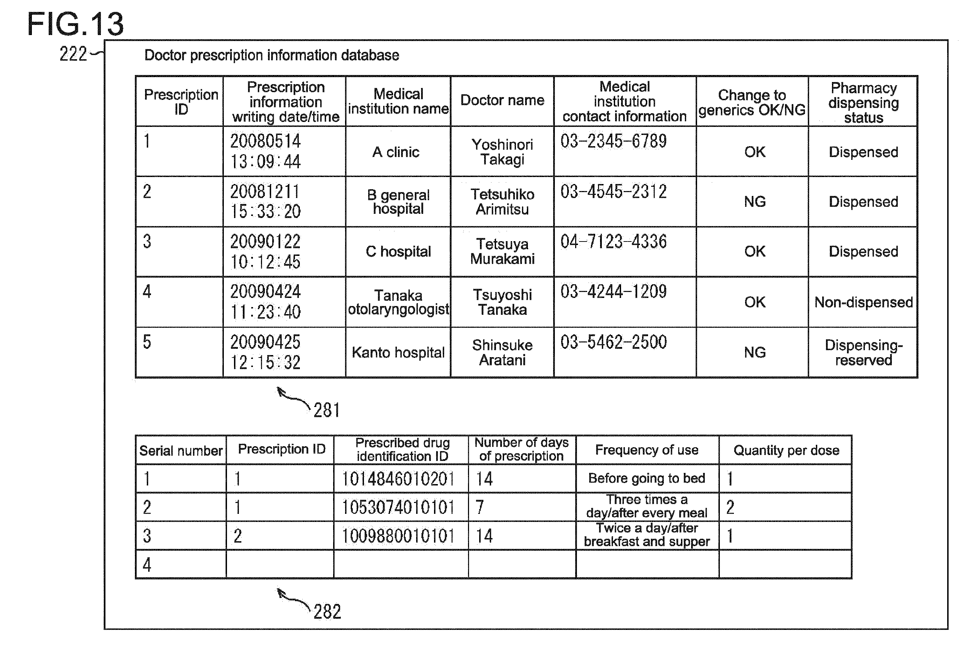

Further, for example, as shown in Fig. 13, the doctor prescription information database 222 of Fig. 10 includes a prescription history index table 281 and a prescription history information table 282.

The prescription history index table 281 includes a prescription history index, which is information on a prescription issued by a doctor at each hospital. Each prescription history index includes "prescription ID", "prescription information writing date/time", "medical institution name", "doctor name", "medical institution contact information", "change to generics OK/NG", and "pharmacy dispensing status".

"Prescription ID" is an ID assigned by the data server 91 in order to identify each prescription, and "medical institution name", "doctor name", and "medical institution contact information" are the name of a medical institution, which issues a prescription, a doctor name, and contact information of the medical institution, respectively.

Further, "change to generics OK/NG" is information showing if a doctor allows generics or not as an alternative drug for a prescribed drug, and "pharmacy dispensing status" is information showing the dispensing status of a drug prescribed based on a prescription at a pharmacy.

For example, in a case where a prescribed drug has been actually dispensed at a pharmacy, "pharmacy dispensing status" is "dispensed", and, in a case where a patient has reserved dispensing but a pharmacy has not dispensed, "pharmacy dispensing status" is "dispensing-reserved". Further, in a case where a patient has not reserved dispensing and a drug has not been actually dispensed, "pharmacy dispensing status" is "non-dispensed".

The prescription history information table 282 includes prescription history information, which is information on a prescription, for each prescription identified by a prescription ID in the prescription history index table 281. The prescription history information includes "serial number", "prescription ID", "prescribed drug identification ID", "number of days of prescription", "frequency of use", and "quantity per dose".

Here, "prescription ID" in the prescription history information is the same as "prescription ID" in the prescription history index of the prescription history index table 281. Therefore, the prescription history index and the prescription history information, which include the same prescription ID, include information on the same prescription.

Further, "prescribed drug identification ID" is an ID identifying a drug prescribed based on a prescription identified by the prescription ID, and is, for example, an ID used for a public database such as Standard Drug Master (Hyoujun Iyakuhin Master), or the like.

The prescription history index table 281 and the prescription history information table 282 are updated every time a doctor issues a prescription and the in-hospital system 11 supplies information on the prescription.

### [About pharmacist dispensing information database]

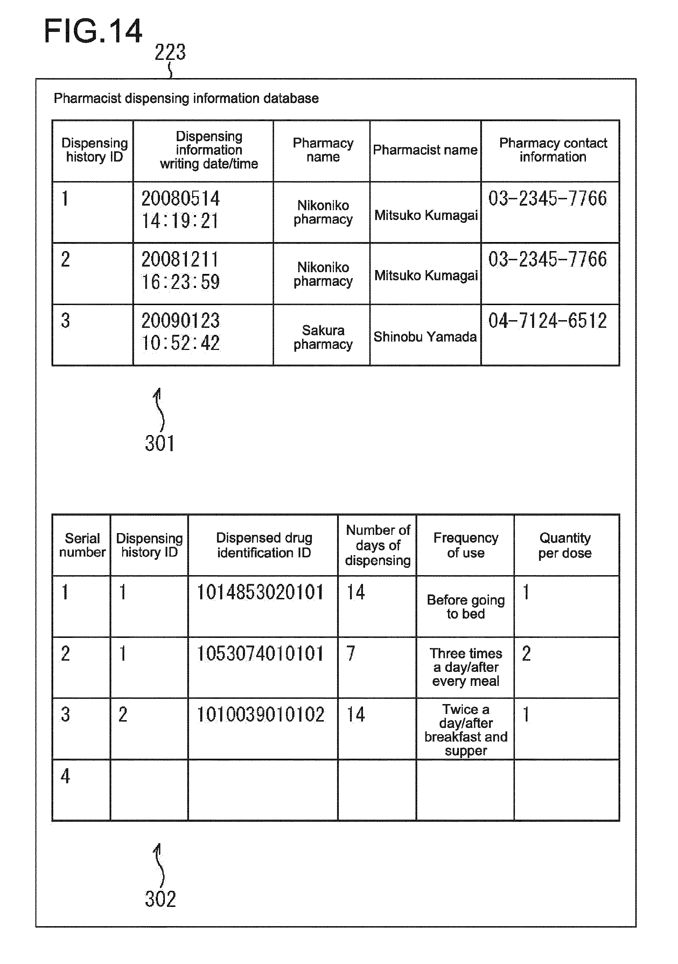

Further, for example, as shown in Fig. 14, the pharmacist dispensing information database 223 of Fig. 10 includes a dispensing history index table 301 and a dispensing history information table 302. The dispensing history index table 301 and the dispensing history information table 302 include information on a drug dispensed based on a prescription issued by a doctor.

Specifically, the dispensing history index table 301 includes a dispensing history index, which is information on dispensing history based on each prescription. The dispensing history index for each prescription in the dispensing history index table 301 includes "dispensing history ID", "dispensing information writing date/time", "pharmacy name", "pharmacist name", and "pharmacy contact information".

Here, "dispensing history ID" is an ID identifying each dispensing history index, and "pharmacy name", "pharmacist name", and "pharmacy contact information" are information showing the name of a pharmacy at which a drug is dispensed, the name of a pharmacist who dispensed a drug, and the telephone number of a pharmacy at which a drug is dispensed, respectively.

Further, the dispensing history information table 302 includes dispensing history information showing dispensing history of a drug, which is created for each drug obtained as the result of dispensing corresponding to a dispensing history index in the dispensing history index table 301.

Each dispensing history information in the dispensing history information table 302 includes "serial number", "dispensing history ID", "dispensed drug identification ID", "number of days of dispensing", "frequency of use", and "quantity per dose".

Here, "dispensing history ID" in the dispensing history information shows to which dispensing history index in the dispensing history index table 301 the dispensing history information corresponds. Further, "dispensed drug identification ID" is an ID identifying a dispensed drug dispensed corresponding to the dispensing history index, and is, for example, an ID used for a public database such as Standard Drug Master (Hyoujun Iyakuhin Master), or the like.

For example, because the dispensing history information having "serial number" of "1" and the dispensing history information having "serial number" of "2" include the same dispensing history ID "1", the dispensing history information is information corresponding to a dispensing history index including the dispensing history ID "1" of the dispensing history index table 301. In this example, two drugs identified by the dispensed drug identification IDs "1014853020101" and "1053074010101" are dispensed as the result of dispensing based on one prescription corresponding to a dispensing history index identified by the dispensing history ID "1".

Note that the drug prescribing history information table 153 of Fig. 6, the drug dispensing history information table 182 of Fig. 8, the drug stock information table 183 of Fig. 9, the adverse effect information table 252 of Fig. 12, the prescription history information table 282 of Fig. 13, and the dispensing history information table 302 of Fig. 14 include, as information identifying a drug, "prescribed drug ID", "dispensed drug ID", "drug ID", "ID of drug that generates adverse effect", "prescribed drug identification ID", and "dispensed drug identification ID", respectively.

A previously-determined common ID is used as an ID identifying a drug. That is, in a case where the IDs are the same, the same drug is identified by the ID. Note that, in a case where the name of a drug identified by an ID is a general name, a plurality of unique drugs may sometimes be identified by the ID.

Further, "frequency of use" of the dispensing history information table 302 is information indicating frequency of use of a dispensed drug, i.e., the number of times of administration of a drug for a day, and the administration timing.

The dispensing history index table 301 and the dispensing history information table 302 in the pharmacist dispensing information database 223 are updated every time a prescribed drug is dispensed at a pharmacy.

### [About return-visit reservation information database]

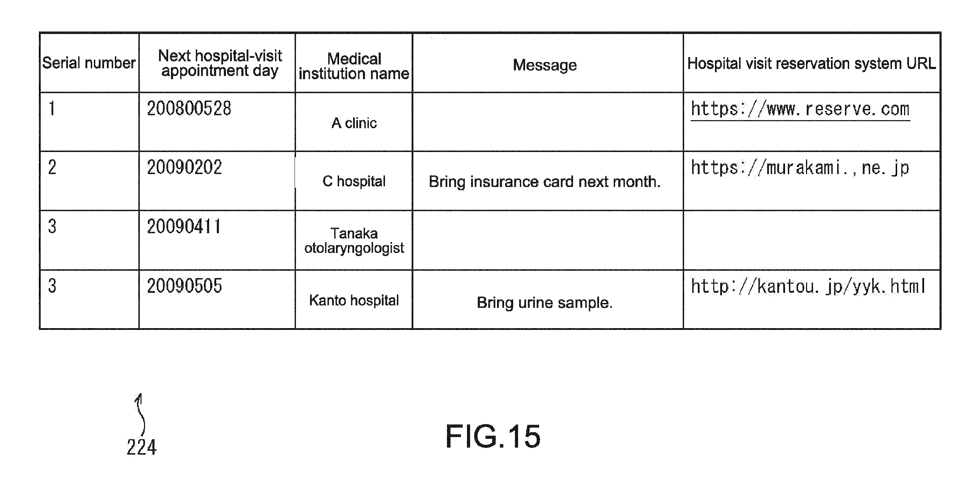

As shown in for example Fig. 15, the return-visit reservation information database 224 of Fig. 10 includes return-visit reservation information, i.e., information on return-visit reservation of a patient at a hospital. Each return-visit reservation information includes "serial number", "next hospital-visit appointment day", "medical institution name", "message", and "hospital visit reservation system URL".

Here, "message" includes information on a message from a hospital side to a patient side, e.g., what a patient brings at the next hospital visit. Further, "hospital visit reservation system URL" includes information on a web page access destination in a case of making a hospital visit reservation by using a web page or the like.

The return-visit reservation information database 224 is updated every time a patient makes a return-visit reservation at a hospital or the like.

### [About dispensing reservation information table]

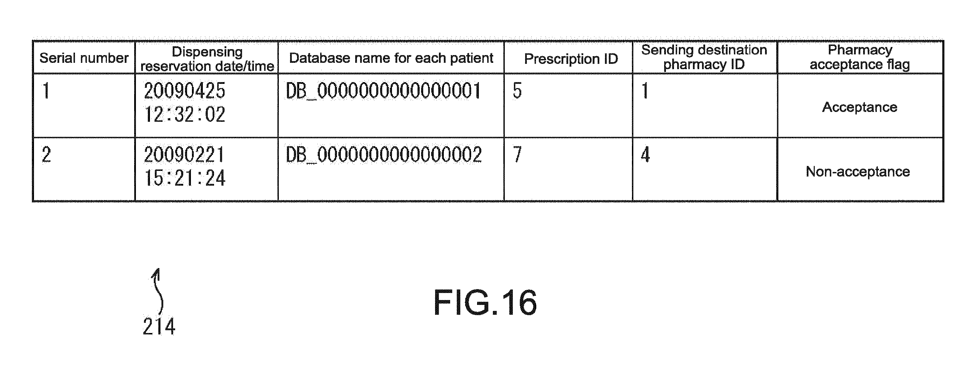

Further, for example, as shown in Fig. 16, the dispensing reservation information table 214 of Fig. 10 includes dispensing reservation information, i.e., information on drug dispensing reservation prescribed by a doctor to a patient. Each piece of dispensing reservation information includes "serial number", "dispensing reservation date/time", "database name for each patient", "prescription ID", "sending destination pharmacy ID", and "pharmacy acceptance flag".

Here, "database name for each patient" includes information for identifying the target patient of dispensing reservation information. Specifically, for example, "database name for each patient" includes information indicating the name of the patient database 212 for each patient.

Further, "prescription ID" is the same as the prescription ID in the prescription history index and the prescription history information of Fig. 13, and is information for identifying a patient's prescription. Further, "sending destination pharmacy ID" is information indicating a prescribed drug dispensing reservation destination. "Pharmacy acceptance flag" is information indicating if a prescribed drug dispensing reservation destination pharmacy accepts a prescribed drug dispensing reservation or not. For example, the pharmacy acceptance flag "acceptance" indicates that dispensing is accepted, and the pharmacy acceptance flag "no acceptance" indicates that dispensing is not accepted.

The dispensing reservation information table 214 is updated every time a patient makes a prescribed drug dispensing reservation.

### <About behaviors of information processing system>

### [About reference to electric medication notebook, etc. at hospital]

Next, behaviors of the respective apparatuses in the information processing system will be described.

For example, a patient is capable of confirming information on a drug that he administers and the like by using the in-hospital system 11 in a hospital.

Specifically, when an examination or the like is executed at a hospital, a patient passes the IC card 14 that he has over the identification information reader 31. When the IC card 14 comes close to the identification information reader 31 by an enough distance, the IC card 14 wirelessly communicates with the identification information reader 31. That is, the identification information reader 31 retrieves patient's identification information from the IC card 14, and supplies the identification information to the in-hospital device 32.

The electric medication notebook reference section 41 of the in-hospital device 32 causes the communication section 45 to send the identification information from the identification information reader 31 to the data server 91 via the communication system 16, and requests to send information in the patient basic information database 221 to the pharmacist dispensing information database 223.

The data server 91 receives identification information sent from the in-hospital device 32, then retrieves information requested by the in-hospital device 32, and sends information obtained as a result to the in-hospital device 32.

That is, with reference to the identification information/patient correspondence database 211, the data server 91 retrieves database name for each patient identified by the received identification information. Then, the data server 91 extracts information in the patient basic information database 221 to the pharmacist dispensing information database 223 from the patient database 212 of the retrieved database name for each patient, and sends the extracted information to the in-hospital device 32 via the communication system 16. More specifically, for example, information extracted as necessary from the patient basic information table 251, the adverse effect information table 252, the prescription history index table 281, the prescription history information table 282, the dispensing history index table 301, and the dispensing history information table 302, are sent to the in-hospital device 32.

In a case where the communication section 45 of the in-hospital device 32 receives information in the patient basic information database 221 to the pharmacist dispensing information database 223 from the data server 91, the electric medication notebook reference section 41 process the information as necessary, and causes the display section 46 to display necessary information. As a result, a patient or a doctor is capable of confirming fundamental information on a patient himself, information on prescription in the past examination, information on a prescribed drug and dispensing history of the prescribed drug, and the like, and it is possible to use the information for examination and the like.

Note that information in the patient basic information database 221 to the pharmacist dispensing information database 223 may be displayed on the in-hospital receipt computer 33 other than the in-hospital device 32.

### [About writing electric prescription]

A doctor finishes examination at a hospital, and then issues a prescription to a patient as necessary. Further, a doctor or the like operates the in-hospital receipt computer 33 to thereby input information on the issued prescription and a prescribed drug, and instructs to update the prescription issued history information table 152 and the drug prescribing history information table 153 in the in-hospital database 47.

Then, the in-hospital receipt computer 33 adds new prescription issued history information to the prescription issued history information table 152 in response to the operation by the doctor or the like, and adds new drug prescribing history information to the drug prescribing history information table 153.

As described above, information on new prescription is recorded in the in-hospital database 47. Then, the in-hospital receipt computer 33 is capable of outputting an application form for a medical expenses statement and demanding a medical expenses statement online based on the prescription issued history information and the drug prescribing history information.

Further, if the in-hospital database 47 is updated, a doctor or the like operates the in-hospital device 32 to thereby write an electric prescription, i.e., instructs to update the doctor prescription information database 222 in the prescription/dispensing database 101. At this time, a patient passes the IC card 14 over the identification information reader 31, and the identification information reader 31 retrieves identification information from the IC card 14 and supplies the identification information to the in-hospital device 32.

Then, the communication section 45 of the in-hospital device 32 sends the identification information supplied from the identification information reader 31 to the data server 91, and requests to send the patient basic information database 221. With reference to the identification information/patient correspondence database 211, the data server 91 identifies the database name for each patient corresponding to the identification information received from the in-hospital device 32. Further, the data server 91 obtains the patient basic information database 221 in the patient database 212 of the database name for each patient, and sends the patient basic information database 221 to the in-hospital device 32.

If the communication section 45 of the in-hospital device 32 receives the patient basic information database 221 from the data server 91, the electric prescription writing section 42 identifies a patient by using the patient basic information database 221 with reference to the in-hospital database 47 of the in-hospital receipt computer 33. For example, the electric prescription writing section 42 retrieves patient basic information having the number same as the insurer number in the patient basic information database 221 received from the patient basic information table 151, to thereby identify a patient.

Further, the electric prescription writing section 42 obtains prescription issued history information and drug prescribing history information on the identified patient from the in-hospital receipt computer 33, and controls the communication section 45 to send necessary information out of the information to the data server 91. At this time, the electric prescription writing section 42 also causes the data server 91 to send information such as a medical institution name, the name of a doctor who issues the prescription, and medical institution contact information, as necessary.

For example, prescription issued history information including a patient number of a identified patient is extracted from the prescription issued history information table 152, and drug prescribing history information having the prescription number same as that of the extracted prescription issued history information is extracted from the drug prescribing history information table 153, which are sent to the data server 91. More specifically, "change to generics OK/NG" is extracted from the prescription issued history information of Fig. 5, and "prescribed drug ID", "number of days of prescription", "frequency of use", and "quantity per dose" are extracted from the drug prescribing history information of Fig. 6, which are sent to the data server 91.

The data server 91 receives respective information in the prescription issued history information and the like from the in-hospital device 32, and then the update section 103 adds a new prescription history index to the prescription history index table 281 of Fig. 13.

At this time, the update section 103 stores an unused unique ID in "prescription ID" of the new prescription history index, stores the present time in "prescription information writing date/time", and stores the respective information received from the in-hospital device 32 in other fields. For example, "change to generics OK/NG" information extracted from the prescription issued history information of Fig. 5 is stored in "change to generics OK/NG" of the prescription history index.

Further, similar to the prescription history index table 281, the update section 103 adds new prescription history information to the prescription history information table 282 of Fig. 13. At this time, the update section 103 provides "serial number" to the new prescription history information, and stores a prescription ID of the corresponding prescription history index in "prescription ID".

Further, the update section 103 stores information on "prescribed drug ID", "number of days of prescription", "frequency of use", and "quantity per dose" of the drug prescribing history information received from the in-hospital device 32 in "prescribed drug identification ID", "number of days of prescription", "frequency of use", and "quantity per dose" of the new prescription history information.

In this manner, information on the prescription recorded in the in-hospital system 11 is supplied to the data server 91, and the doctor prescription information database 222 is updated. As a result, it is possible to share information on each patient's prescription.

### [About dispensing reservation]

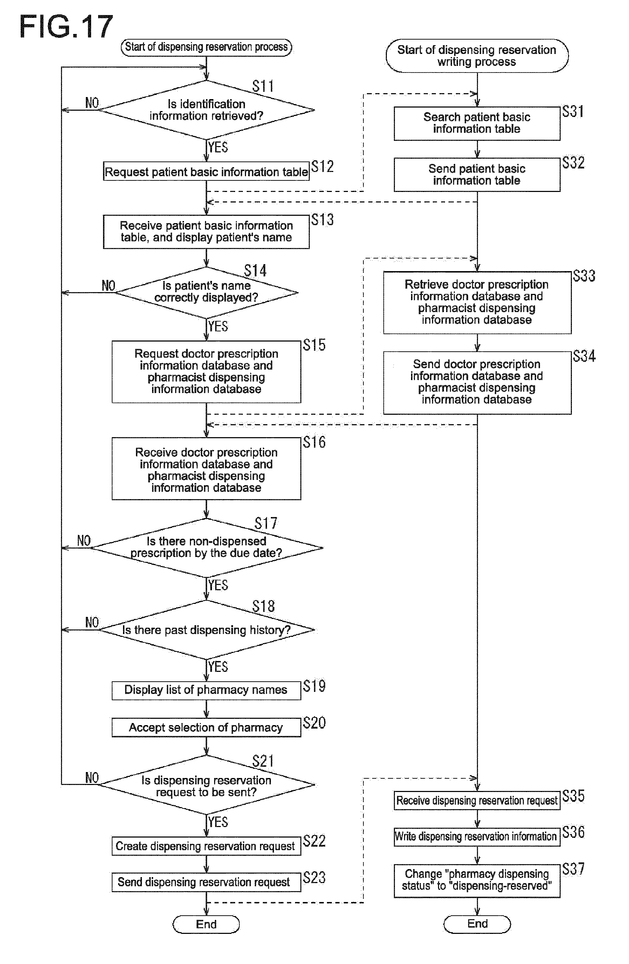

Further, a patient is capable of making a drug dispensing reservation of a drug, which is prescribed based on a prescription at a hospital after examination at the hospital is finished. Hereinafter, with reference to the flowchart of Fig. 17, dispensing reservation process and dispensing reservation writing process executed by the in-hospital device 32 and the data server 91 in such a case will be described.

For example, at a time when the in-hospital device 32 starts a dispensing reservation process, the in-hospital device 32 displays a message e.g., "pass IC card", for urging a patient to pass the IC card 14 over the identification information reader 31 in the case of dispensing reservation. A patient watches the message, and passes the IC card 14 that he has over the identification information reader 31.

In Step S11, the dispensing reservation section 43 of the in-hospital device 32 determines if the IC card 14 is passed over the identification information reader 31 and identification information is retrieved or not. For example, in a case where identification information, which is retrieved from the IC card 14, is supplied from the identification information reader 31 to the dispensing reservation section 43, it is determined that identification information is retrieved.

In Step S11, in a case where it is determined that identification information is retrieved, the process returns to Step S11, and the above-mentioned process is repeated.

Meanwhile, in a case where it is determined that identification information is retrieved in Step S11, the communication section 45 sends the retrieved identification information to the data server 91, and requests to send a patient basic information table in Step S12.

Then, in Step S31, the data server 91 searches a patient basic information table. That is, in a case where the communication section 102 receives identification information sent from the in-hospital device 32, the data server 91 refers to the identification information/patient correspondence database 211 in response to the request from the in-hospital device 32, and identifies a database name for each patient in association with identification information. Then, the data server 91 extracts the patient basic information table from the patient basic information database 221, which constitutes the patient database 212 of the identified name.

In Step S32, the communication section 102 of the data server 91 sends the extracted patient basic information table to the in-hospital device 32.

In Step S13, the communication section 45 of the in-hospital device 32 receives patient basic information table sent from the data server 91, and the dispensing reservation section 43 causes the display section 46 to display the patient's name based on the patient basic information table.

For example, the dispensing reservation section 43 identifies patient basic information, which includes an insurer number same as the insurer number in the patient basic information table, based on the patient basic information table 151 in the in-hospital database 47. Then, the dispensing reservation section 43 causes the display section 46 to display the patient's name in the identified patient basic information.

As a result, a message, e.g., "are you Taro Tanaka?", for asking the patient's name is displayed on the in-hospital device 32. The patient watches this message, and operates the in-hospital device 32 to thereby input if his name is correctly displayed.

In Step S14, the dispensing reservation section 43 determines if the patient's name is correctly displayed or not based on the operation input by a patient. In a case where it is determined that the patient's name is not correctly displayed in Step S14, the process returns to Step S11, and the above-mentioned process is repeated. In this case, the in-hospital device 32 displays a message, e.g., "come to window".

Meanwhile, if it is determined that the name is correctly displayed in Step S14, the process proceeds to Step S15. In Step S15, the communication section 45 sends the identification information, which is retrieved from the IC card 14 of the patient, to the data server 91, and requests to send a doctor prescription information database and a pharmacist dispensing information database.

Then, in Step S33, the data server 91 retrieves the doctor prescription information database and the pharmacist dispensing information database.

That is, the data server 91 receives identification information sent from the in-hospital device 32, and then identifies a database name for each patient in association with the received identification information with reference to the identification information/patient correspondence database 211 in response to the request from the in-hospital device 32. Then, the data server 91 extracts the doctor prescription information database 222 and the pharmacist dispensing information database 223 based on the patient database 212 of the identified name.

In Step S34, the communication section 102 of the data server 91 sends the extracted doctor prescription information database and pharmacist dispensing information database to the in-hospital device 32.

In Step S16, the communication section 45 receives the doctor prescription information database and the pharmacist dispensing information database sent from the data server 91.

In Step S17, the dispensing reservation section 43 determines if there is a non-dispensed prescription by the due date or not.

For example, in general, a prescription is valid for four days after the day of issue. In view of this, the dispensing reservation section 43 searches the prescription history index table 281 constructing the received doctor prescription information database 222 for a prescription history index satisfying the following condition. That is, the dispensing reservation section 43 retrieves a prescription history index whose "prescription information writing date/time" is within four days from the present date/time and whose "pharmacy dispensing status" is "non-dispensed".

A prescription identified by a prescription ID of the thus retrieved prescription history index is valid and a prescribed drug is not dispensed. So it is determined that such a prescription is a valid and non-dispensed prescription.

In a case where it is determined that there is no valid and non-dispensed prescription in Step S17, the process returns to Step S11, and the above-mentioned process is repeated. At this time, for example, the in-hospital device 32 displays a message, e.g., "no prescription", indicating that there is no prescription, which requires dispensing reservation.

Meanwhile, in a case where it is determined that there is a valid non-dispensed prescription in Step S17, the dispensing reservation section 43 determines if there is past dispensing history or not in Step S18. Specifically, in a case where the dispensing history index table 301 in the pharmacist dispensing information database 223, which is received from the data server 91, includes at least one dispensing history index, it is determined that there is past dispensing history.

In a case where it is determined that there is no past dispensing history in Step S18, the process returns to Step S11, and the above-mentioned process is repeated. At this time, for example, the in-hospital device 32 displays a message, e.g., "no pharmacy data", indicating that there is no history of a pharmacy to which dispensing is requested in the past.

Meanwhile, if it is determined that there is a past dispensing history in Step S18, the dispensing reservation section 43 causes the display section 46 to display a list of pharmacy names in Step S19.

That is, the dispensing reservation section 43 refers to dispensing history indexes in the dispensing history index table 301 in the received pharmacist dispensing information database 223, and extracts "pharmacy name" of the dispensing history indexes. Then, the dispensing reservation section 43 creates a list of the extracted pharmacy names, and causes the in-hospital device 32 to display the list. The thus-displayed list of pharmacy names is a list of names of pharmacies to which dispensing is requested by a patient in the past.

In Step S20, the dispensing reservation section 43 accepts selection of a pharmacy, to which dispensing of a prescribed drug is to be requested this time. In the case where the list of pharmacy names is displayed, a patient operates the in-hospital device 32, and specifies a pharmacy, to which dispensing of the prescribed drug is to be requested this time, from the displayed list.

Note that a pharmacy, to which dispensing is to be requested, is not limited to a pharmacy, to which dispensing was requested in the past, but may be a pharmacy near the hospital or the patient's home, or may be a pharmacy retrieved based on word search or the like. In such cases, a list or the like of those pharmacies is displayed, and dispensing is requested to a pharmacy specified by a patient.

In Step S21, the dispensing reservation section 43 determines whether to send a dispensing reservation request to request dispensing reservation or not. For example, in a case where a patient selects the name of a pharmacy, to which dispensing is to be requested, from the list of pharmacy names, it is determined that a dispensing reservation request is sent. Further, for example, in a case where a pharmacy name is not selected after a predetermined time has passed, it is determined that a dispensing reservation request is not sent.

If it is determined that a dispensing reservation request is not sent in Step S21, the process returns to Step S11, and the above-mentioned process is repeated.

Meanwhile, if it is determined that a dispensing reservation request is sent in Step S21, the dispensing reservation section 43 creates a dispensing reservation request in Step S22.

Specifically, the dispensing reservation section 43 creates a dispensing reservation request, which includes identification information retrieved from the IC card 14 of a patient, a prescription ID identifying a prescription to be processed, and a sending destination pharmacy ID identifying a pharmacy selected by a patient.

Here, the prescription ID in the dispensing reservation request is the prescription ID in the prescription history index of the valid non-dispensed prescription, which is retrieved in Step S17. Further, the in-hospital device 32 is configured to obtain a sending destination pharmacy ID, which identifies a pharmacy based on the name of each pharmacy.

Note that the dispensing reservation request may additionally include information showing if a patient desires generics or not, and information on a drug shape (type of drug) that a patient desires.

If the dispensing reservation request includes information showing if a patient desires generics or not, and information on a drug shape that a patient desires, a pharmacist, who is requested to dispense a drug based on the dispensing reservation request, is capable of knowing what a patient desires with respect to drug, and can start dispensing immediately.

For example, a pharmacy side cannot know detailed hope (if patient desires generics, if patient desires liquid drug, etc.) of a patient about a drug only based on an ID (for example, ID identifying general name of drug) identifying a drug of which dispensing is reserved. Because of this, if a pharmacy dispenses a drug beforehand in a state where the pharmacy only knows an ID identifying a drug, there may be a situation where it is revealed that the dispensed drug is different from a drug that a patient desires when the pharmacy gives the drug to the patient.

To the contrary, if a dispensing reservation request includes information showing patient's detailed hope (e.g., if patient desires generics) about drug, a pharmacy side is capable of previously preparing a drug that a patient desires, and it is possible to improve convenience for a patient.

In Step S23, the communication section 45 sends the created dispensing reservation request to the data server 91, and the dispensing reservation process is finished. Further, if the dispensing reservation request is sent, the display section 46 displays a message, e.g., "dispensing reservation is done", showing that dispensing reservation is done.

If the dispensing reservation request is sent to the data server 91, in Step S35, the communication section 102 of the data server 91 receives the dispensing reservation request sent from the in-hospital device 32.

Then, in Step S36, the update section 103 of the data server 91 writes dispensing reservation information in the dispensing reservation information table 214 based on the received dispensing reservation request.

Specifically, the update section 103 provides a field of new dispensing reservation information in the dispensing reservation information table 214 of Fig. 16, and stores a numerical value, which is obtained by incrementing the largest value of serial numbers assigned to other dispensing reservation information, in "serial number" of the dispensing reservation information. Further, the update section 103 stores the present date/time in "dispensing reservation date/time" of the new dispensing reservation information, and stores the database name for each patient, which is identified based on identification information in the dispensing reservation request, in "patient database name" of the dispensing reservation information.

Further, the update section 103 stores a prescription ID and a sending destination pharmacy ID, which are in the dispensing reservation request, in "prescription ID" and "sending destination pharmacy ID" in the dispensing reservation information. Note that "pharmacy acceptance flag" in the dispensing reservation information indicates a state where nothing is stored. If the dispensing reservation request includes information showing that a patient desires generics or not and information showing a desired drug shape, the information is added to the dispensing reservation information.

Note that it has been described that the dispensing reservation request may include information showing if a patient desires generics or not and information on a desired drug shape, which are input by operating the in-hospital device 32 by a doctor or the like. Alternatively, the data server 91 may create the information.
In this case, the data server 91 creates information showing if a patient desires generics or not and information on a desired drug shape with reference to the patient basic information table 251, for example, and creates dispensing reservation information including the information.

In Step S37, the update section 103 changes "pharmacy dispensing status" in a prescription history index of a prescription of a patient, of which dispensing reservation is done, in the prescription history index table 281 of Fig. 13, to "dispensing-reserved". The dispensing reservation writing process is thus finished.

That is, the update section 103 refers to the identification information/patient correspondence database 211 of Fig. 11, and identifies database name for each patient, which is in relation with identification information in the dispensing reservation request. Further, the update section 103 updates the prescription history index table 281 of the patient database 212 of the identified name.

Specifically, the update section 103 retrieves a prescription history index including a prescription ID in the dispensing reservation request, out of prescription history indexes in the prescription history index table 281 of Fig. 13. The update section 103 changes "pharmacy dispensing status" in the retrieved prescription history index from "non-dispensed" to "dispensing-reserved".

Note that, in the dispensing reservation writing process, the respective processes are executed as necessary and as required. For example, after the process of Step S32, if it is determined that a name is not displayed correctly in Step S14, the processes of Step S31 and Step S32 are executed again.

As described above, in a state where information on a prescription is recorded in the data server 91, the in-hospital device 32 obtains information on a patient from the data server 91 by using identification information of the patient as a key, and sends a dispensing reservation request to the data server 91. Further, the data server 91 receives the dispensing reservation request, then updates a dispensing reservation information table based on the dispensing reservation request, and reserves dispensing.

According to the information processing system of Fig. 1, the in-hospital device 32 sends a dispensing reservation request to the data server 91, and the data server 91 updates a dispensing reservation information table. Because of this, after a patient is examined at a hospital, the patient is capable of reserving dispensing to a pharmacy immediately. As described above, information on a prescription is recorded in the data server 91 beforehand, and a patient is capable of reserving dispensing before he goes to a pharmacy. Because of this, it is possible to reduce a patient's waiting time at a pharmacy, and to improve convenience.

### [About acceptance of dispensing]

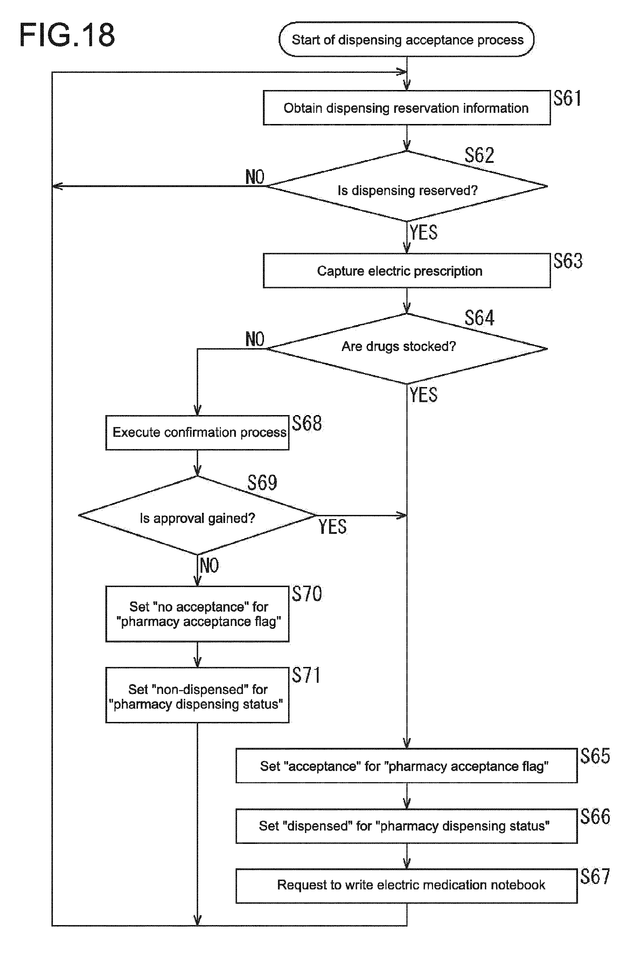

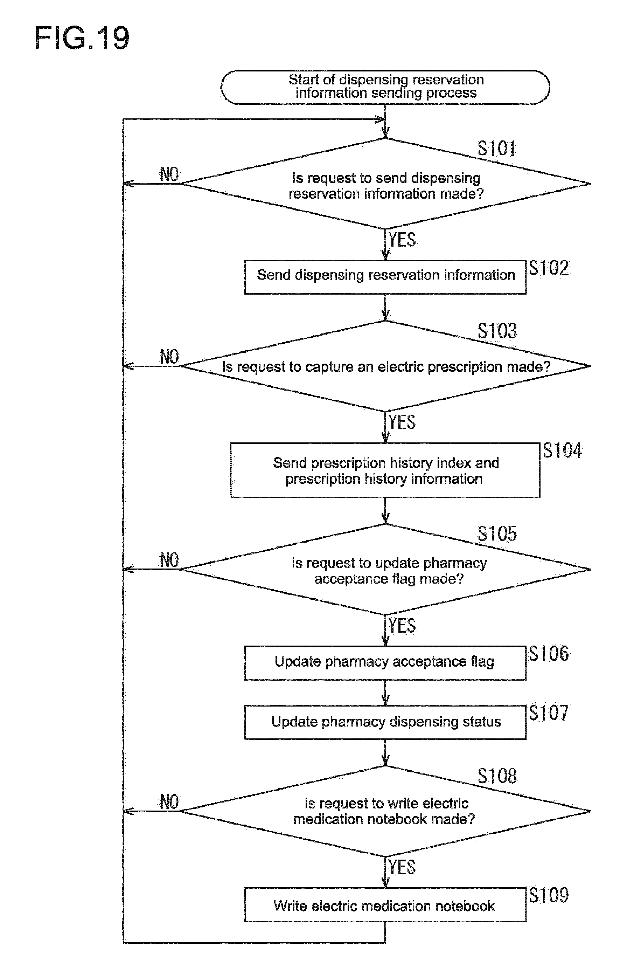

If the data server 91 adds new dispensing reservation information to the dispensing reservation information table 214, it means that a patient reserves dispensing. When dispensing is reserved, the in-pharmacy device 62 at a pharmacy side accepts dispensing reservation, and a pharmacist dispenses a drug. Hereinafter, with reference to flowcharts of Fig. 18 and Fig. 19, processes executed by the in-pharmacy device 62 and the data server 91 when accepting dispensing reservation will be described.

First, with reference to the flowchart of Fig. 18, the dispensing acceptance process executed by the in-pharmacy device 62 will be described.

In Step S61, the dispensing reservation section 74 of the in-pharmacy device 62 obtains dispensing reservation information from the data server 91.

For example, the dispensing reservation section 74 requests the data server 91 to send dispensing reservation information, and then the data server 91 sends, to the in-pharmacy device 62, dispensing reservation information, to which "pharmacy acceptance flag" is not set, and which has a sending destination pharmacy ID same as the pharmacy ID of the request source, out of dispensing reservation information in the dispensing reservation information table 214 of Fig. 16.

That is, the sent dispensing reservation information includes "pharmacy acceptance flag", which indicates neither acceptance nor no acceptance, and includes a sending destination pharmacy ID showing a pharmacy in which the in-pharmacy device 62 is installed. The dispensing reservation section 74 causes the communication section 75 to receive the dispensing reservation information thus sent from the data server 91, to thereby obtain dispensing reservation information.

Note that the in-pharmacy device 62 may regularly accesses the data server 91 to thereby obtain the dispensing reservation information, or the data server 91 may send the dispensing reservation information to the in-pharmacy device 62 every time the data server 91 updates the dispensing reservation information table 214.

In Step S62, the dispensing reservation section 74 determines if a patient has reserved dispensing or not based on presence/absence of obtained dispensing reservation information.

If it is determined that dispensing is not reserved in Step S62, the process returns to Step S61, and the above-mentioned process is repeated.

Meanwhile, if it is determined that dispensing is reserved in Step S62, the dispensing reservation section 74 causes the display section 76 to display a message, e.g., "dispensing reserved", showing that dispensing is reserved, and the process proceeds to Step S63.
Note that the message showing that dispensing is reserved may be displayed on the in-pharmacy receipt computer 63.

In Step S63, the electric prescription capturing section 72 only captures an electric prescription. That is, the electric prescription capturing section 72 extracts a prescription ID from dispensing reservation information including a sending destination pharmacy ID, which shows a pharmacy in which the in-pharmacy device 62 is installed.

Then, the electric prescription capturing section 72 controls the communication section 75 to thereby cause the data server 91 to send an electric prescription capturing request, which includes the extracted prescription ID, to request to send a prescription history index and prescription history information.

Then, the data server 91 sends a prescription history index and prescription history information in response to the electric prescription capturing request. The electric prescription capturing section 72 causes the communication section 75 to receive the prescription history index and prescription history information sent from the data server 91.

Here, the prescription history index and the prescription history information sent from the data server 91 are the prescription history index and the prescription history information, which include the prescription ID in the electric prescription capturing request extracted from the doctor prescription information database 222 of Fig. 13.

In Step S64, the dispensing reservation section 74 determines if a drug, of which dispensing is reserved, is stocked or not, based on "prescribed drug identification ID", "number of days of prescription", "frequency of use", and "quantity per dose" in the obtained prescription history information and based on the drug stock information table 183.

For example, the dispensing reservation section 74 obtains the amount of drugs to be dispensed based on "number of days of prescription", "frequency of use", and "quantity per dose" in the prescription history information. Further, the dispensing reservation section 74 identifies drug stock information including an ID, which is the same as "prescribed drug identification ID" in the prescription history information, from the drug stock information table 183 of Fig. 9 in the in-pharmacy database 77 of the in-pharmacy receipt computer 63.

Then, if the amount of "stock amount" in the identified drug stock information is larger than the amount of drug to be dispensed, the dispensing reservation section 74 determines that drugs are stocked. Note that, if a plurality of drugs are prescribed with one prescription, in a case of stock shortage of at least one drug, it is determined that drugs are not stocked.

Here, if information showing if a patient desires generics or not and information showing a desired drug shape are added to the dispensing reservation information, it is determined if drugs are stocked or not based on the information. For example, if information that a patient desires generics is added to the dispensing reservation information, it is determined if generics for a drug of which dispensing is reserved are stocked or not. Further, for example, in a case where information showing a drug shape that a patient desires is added to the dispensing reservation information, if there is no drug having the drug shape shown in the information, it is determined that the drugs are not stocked.

Note that, if a neighboring pharmacy is capable of providing drugs as necessary, it may be determined if drugs are stocked or not with consideration of the stock status at the neighboring pharmacy. Further, the data server 91 may determine if drugs are stocked or not.

If it is determined that drugs are stocked in Step S64, the dispensing reservation section 74 sets "acceptance" for "pharmacy acceptance flag" of the dispensing reservation information of a drug to be dispensed in the dispensing reservation information table 214 in Step S65.

That is, the dispensing reservation section 74 controls the communication section 75 to thereby cause the data server 91 to send a pharmacy acceptance flag change request, which includes a prescription ID of dispensing reservation information of a drug to be dispensed, and which requests to set "acceptance" for "pharmacy acceptance flag". The data server 91 receives the pharmacy acceptance flag change request, then identifies dispensing reservation information including the prescription ID in the pharmacy acceptance flag change request, from the dispensing reservation information table 214 of Fig. 16, and sets "acceptance" for "pharmacy acceptance flag" of the identified dispensing reservation information.

In Step S66, the dispensing reservation section 74 sets "dispensed" for "pharmacy dispensing status" of the prescription history index of a prescription of a drug to be dispensed in the prescription history index table 281.

That is, the dispensing reservation section 74 controls the communication section 75 to thereby cause the data server 91 to send a pharmacy dispensing status change request, which includes a prescription ID of dispensing reservation information of a drug to be dispensed, and which requests to set "dispensed" for "pharmacy dispensing status". The data server 91 receives the pharmacy dispensing status change request, then identifies a prescription history index including the prescription ID in the pharmacy dispensing status change request from the prescription history index table 281 of Fig. 13, and sets "dispensed" for "pharmacy dispensing status" of the identified prescription history index.

As described above, the pharmacy acceptance flag and the pharmacy dispensing status are updated, and dispensing of a drug is accepted. Then, for example, the display section 76 displays necessary information such as the prescription ID of dispensing reservation information, the name of a drug to be dispensed identified by the prescribed drug identification ID, number of days of prescription, frequency of use, and quantity per dose. A pharmacist watches the display, and is capable of starting to dispense a drug actually.

Further, when a pharmacist dispenses the prescribed drug, the pharmacist operates the in-pharmacy device 62 to thereby input information on the dispensed drug. Then, in Step S67, the electric medication notebook writing section 73 of the in-pharmacy device 62 requests the data server 91 to write an electric medication notebook in response to an operation input by the pharmacist.

For example, the electric medication notebook writing section 73 creates an electric medication notebook writing request, which includes a pharmacy name, a pharmacist name, and pharmacy contact information input by a pharmacist as necessary, and includes a dispensed drug identification ID, number of days of dispensing, frequency of use, and quantity per dose of a dispensed drug. Then, the electric medication notebook writing section 73 controls the communication section 75 to thereby cause the data server 91 to send the electric medication notebook writing request.

Here, the dispensed drug identification ID, the number of days of dispensing, the frequency of use, and the quantity per dose in the electric medication notebook writing request are determined based on "prescribed drug identification ID", "number of days of prescription", "frequency of use", and "quantity per dose" and the like of the prescription history information captured in Step S63, for example.

The data server 91 receives the electric medication notebook writing request, and then updates the pharmacist dispensing information database 223 of Fig. 14 based on the electric medication notebook writing request. That is, the update section 103 adds a new dispensing history index to the dispensing history index table 301 of Fig. 14, and stores an ID, which is not provided to the other dispensing history indexes, in "dispensing history ID" of the dispensing history index.

Then, the update section 103 stores the present date/time in "dispensing information writing date/time" of the dispensing history index, and stores the pharmacy name, the pharmacist name, and the pharmacy contact information, which are in the electric medication notebook writing request, in "pharmacy name", "pharmacist name", and "pharmacy contact information" of the dispensing history index.

Further, the update section 103 adds new dispensing history information in the dispensing history information table 302 of Fig. 14, and stores a new serial number and an ID, which is the same as "dispensing history ID" of the new dispensing history index, in "serial number" and "dispensing history ID" of the dispensing history information, respectively. Further, the update section 103 stores the dispensed drug identification ID, the number of days of dispensing, the frequency of use, and the quantity per dose, which are in the electric medication notebook writing request, in "dispensed drug identification ID", "number of days of dispensing", "frequency of use", and "quantity per dose" of the new dispensing history information.

If a drug is further dispensed, the in-pharmacy receipt computer 63 updates the drug stock information table 183 based on an instruction from the in-pharmacy device 62. That is, the amount of "stock amount" of drug stock information is reduced by the dispensed amount.

Further, after a drug is dispensed, the in-pharmacy device 62 instructs the in-pharmacy receipt computer 63 to update the drug dispensing history information table 182.

Specifically, the in-pharmacy device 62 sends a patient basic information table sending request, which includes a prescription ID identifying a prescription of a dispensed drug, to the data server 91, and obtains the patient basic information table 251 of Fig. 12 from the data server 91. Here, the data server 91 searches the prescription history index table 281 including the received prescription ID to thereby identify the patient database 212, and sends the patient basic information table 251 constituting the patient database 212.

The in-pharmacy device 62 receives the patient basic information table 251 from the data server 91, and then extracts a patient's insurer number from the patient basic information table 251. Then, the in-pharmacy device 62 supplies the dispensed drug identification ID, number of days of dispensing, frequency of use, and quantity per dose of the dispensed drug, and the extracted patient's insurer number to the in-pharmacy receipt computer 63, and instructs to update the drug dispensing history information table 182.

Then, the in-pharmacy receipt computer 63 adds the new drug dispensing history information to the drug dispensing history information table 182 of the in-pharmacy database 77 in response to the instruction from the in-pharmacy device 62, and updates the drug dispensing history information table 182.

Specifically, the in-pharmacy receipt computer 63 stores a new serial number in "serial number" of drug dispensing history information, and stores the present time in "dispensing date/time". Further, with reference to the patient basic information table 181, the in-pharmacy receipt computer 63 identifies a patient's patient number based on the insurer number supplied from the in-pharmacy device 62, and stores the identified patient number in "patient number" of drug dispensing history information.

Further, the in-pharmacy receipt computer 63 stores the dispensed drug identification ID, number of days of dispensing, frequency of use, and quantity per dose, which are supplied from the in-pharmacy device 62, in "dispensed drug ID", "number of days of prescription", "frequency of use", and "quantity per dose" of drug dispensing history information. Then, the in-pharmacy receipt computer 63 stores a drug name and its type, which are identified based on the dispensed drug identification ID of a dispensed drug, in "dispensed drug name" and "type" of drug dispensing history information.

In this manner, information on the newly-dispensed drug is recorded in the in-pharmacy database 77. Then, the in-pharmacy receipt computer 63 is capable of outputting an application form for a medical expenses statement and demanding a medical expenses statement online, based on drug dispensing history information.

Note that the in-pharmacy device 62 may not instruct the in-pharmacy receipt computer 63 to write an electric medication notebook, update the drug stock information table 183, and update the drug dispensing history information table 182. For example, the in-pharmacy device 62 may assist the in-pharmacy receipt computer 63 to input information, and a pharmacist or the like may operate the in-pharmacy receipt computer 63 to thereby write an electric medication notebook and the like.

As described above, the pharmacist dispensing information database 223 and the like are updated, then the process returns to Step S61, and the above-mentioned process is repeated.

Further, if it is determined that drugs are not stocked in Step S64, the dispensing reservation section 74 executes confirmation process in Step S68.

For example, the dispensing reservation section 74 controls the communication section 75 to thereby send a patient basic information table sending request, which includes a prescription ID of the dispensing reservation information obtained in Step S61, to the data server 91, and obtains the patient basic information table 251 of Fig. 12 from the data server 91.

That is, the data server 91 identifies the patient database 212, in which a prescription history index including the prescription ID is recorded, based on the prescription ID in the patient basic information table sending request received from the in-pharmacy device 62. Then, the data server 91 sends the patient basic information table 251, which constitutes the identified patient database 212, to the in-pharmacy device 62.

The dispensing reservation section 74 receives the patient basic information table 251 from the data server 91, then identifies the name and contact information of a patient who reserved dispensing of a drug with reference to the patient basic information table 181 in the in-pharmacy database 77, and causes the display section 76 to display the name and contact information. For example, a patient is identified by searching the patient basic information table 181 for patient basic information including an insurer number in the patient basic information table 251.

The patient's name and contact information are displayed on the in-pharmacy device 62, and then a pharmacist contacts the patient by means of the displayed contact information such as a telephone number or an email address, and confirms if the pharmacist is capable of responding to the patient's desire or not. For example, a pharmacist directly calls a patient, and confirms the patient "only drug A is dispensed at a later date, OK?", "generics for drug B are not stocked, and the original drug B is dispensed. OK?", "you desire powder, but is tablet OK?", etc.

Then, pharmacist gains approval from a patient, and then operates the in-pharmacy device 62 to input the confirmation result. That is, whether approval from a patient is gained or not is input. At this time, a pharmacist operates the in-pharmacy device 62, and inputs as necessary that a patient desires powder as a drug shape but tablet is permitted, and the like.

Note that, the in-pharmacy device 62 may send inquiries to the in-hospital device 32 via the data server 91, or may send inquiries to the mobile terminal device 15 of a patient, to thereby confirm if the pharmacist is capable of responding to the patient's desire or not.

In Step S69, the dispensing reservation section 74 determines if approval from a patient is gained or not, based on operation input in the in-pharmacy device 62 by a pharmacist.

If it is determined that approval from a patient is gained in Step S69, the process proceeds to Step S65, and the above-mentioned Step S65 to Step S67 are processed. At this time, information input in Step S68 is used as necessary. For example, in a case where a patient desires powder as a drug shape but permits tablet, and information thereon is input, a drug of which dispensing is reserved having a tablet shape is dispensed. Then, an electric medication notebook writing request including the dispensed drug identification ID of the drug is sent to the data server 91.

Meanwhile, if it is determined that approval is not gained in Step S69, the dispensing reservation section 74 sets "no acceptance" for "pharmacy acceptance flag" of dispensing reservation information of a drug, of which dispensing is reserved, in the dispensing reservation information table 214 in Step S70.

That is, the dispensing reservation section 74 controls the communication section 75 to thereby send a pharmacy acceptance flag change request, which includes a prescription ID of dispensing reservation information of a drug of which dispensing is reserved, and which requests to set "no acceptance" for "pharmacy acceptance flag", to the data server 91.

The communication section 102 receives the pharmacy acceptance flag change request, and then the update section 103 identifies dispensing reservation information, which includes the prescription ID in the pharmacy acceptance flag change request, from the dispensing reservation information table 214 of Fig. 16, and sets "no acceptance" for "pharmacy acceptance flag" of the identified dispensing reservation information.

In Step S71, the dispensing reservation section 74 sets "non-dispensed" for "pharmacy dispensing status" of the prescription history index of a prescription of a drug, of which dispensing is reserved, in the prescription history index table 281.

That is, the dispensing reservation section 74 controls the communication section 75 to thereby send a pharmacy dispensing status change request, which includes a prescription ID of dispensing reservation information of a drug of which dispensing is reserved, and which requests to set "non-dispensed" for "pharmacy dispensing status", to the data server 91. The data server 91 receives the pharmacy dispensing status change request, then identifies a prescription history index, which includes the prescription ID in the pharmacy dispensing status change request, from the prescription history index table 281 of Fig. 13, and sets "non-dispensed" for "pharmacy dispensing status" of the identified prescription history index.

In this manner, the dispensing reservation information table 214 and the prescription history index table 281 are updated, then the process returns to Step S61, and the above-mentioned process is repeated.

Note that, in a case where "no acceptance" is set for "pharmacy acceptance flag" of dispensing reservation information of the dispensing reservation information table 214, the data server 91 may notify the in-hospital device 32, the mobile terminal device 15 of a patient, and the like, that dispensing reservation is not accepted.

As described above, the in-pharmacy device 62 regularly determines if dispensing is reserved, and, if dispensing is reserved, the in-pharmacy device 62 confirms stock of drugs, and accepts dispensing reservation.

In this manner, the in-pharmacy device 62 regularly accesses the data server 91, determines if dispensing is reserved, and accepts dispensing reservation. Because of this, a pharmacist is capable of starting to dispense a drug before a patient visits a pharmacy. As a result, patient's waiting time is reduced, customer satisfaction may be increased, and rotation of customers may be increased.

Next, with reference to the flowchart of Fig. 19, dispensing reservation information sending process, which is executed by the data server 91 when the in-pharmacy device 62 is executing the dispensing acceptance process of Fig. 18, will be described.

In Step S101, the data server 91 determines if the in-pharmacy device 62 requests to send dispensing reservation information or not. If it is determined that the in-pharmacy device 62 does not request to send dispensing reservation information in Step S101, the process returns to Step S101, and the above-mentioned process is repeated.

Meanwhile, if it is determined that the in-pharmacy device 62 requests to send dispensing reservation information in Step S101, the communication section 102 of the data server 91 sends the requested dispensing reservation information to the in-pharmacy device 62 in Step S102. For example, dispensing reservation information, to which "pharmacy acceptance flag" is not set, and which has sending destination pharmacy ID same as the pharmacy ID of the request source, is sent to the in-pharmacy device 62 out of dispensing reservation information in the dispensing reservation information table 214 of Fig. 16.

In Step S103, the data server 91 determines if the in-pharmacy device 62 requests to capture an electric prescription. For example, if the in-pharmacy device 62 sends an electric prescription capturing request, it is determined that the in-pharmacy device 62 requests to capture an electric prescription.

If it is determined that the in-pharmacy device 62 does not request to capture an electric prescription in Step S103, the process returns to Step S101, and the above-mentioned process is repeated.

Meanwhile, if it is determined that the in-pharmacy device 62 requests to capture an electric prescription in Step S103, the communication section 102 receives the sent electric prescription capturing request, and the process proceeds to Step S104.

In Step S104, the data server 91 extracts a prescription history index and prescription history information, each of which includes the prescription ID in the electric prescription capturing request, from the doctor prescription information database 222 of Fig. 13. Then, the communication section 102 sends the prescription history index and the prescription history information to the in-pharmacy device 62.

In Step S105, the data server 91 determines if a request to update a pharmacy acceptance flag is made or not. For example, if the in-pharmacy device 62 sends a pharmacy acceptance flag change request, it is determined that a request to update a pharmacy acceptance flag is made.

If it is determined that a request to update a pharmacy acceptance flag is not made in Step S105, the process returns to Step S101, and the above-mentioned process is repeated.

Meanwhile, if it is determined that a request to update a pharmacy acceptance flag is made in Step S105, the communication section 102 receives the pharmacy acceptance flag change request sent from the in-pharmacy device 62, and the process proceeds to Step S106.

In Step S106, the update section 103 updates the pharmacy acceptance flag in response to the pharmacy acceptance flag change request. That is, the update section 103 identifies dispensing reservation information, which includes the prescription ID in the pharmacy acceptance flag change request, from the dispensing reservation information table 214 of Fig. 16, and sets "pharmacy acceptance flag" of the identified dispensing reservation information.

Specifically, if a pharmacy acceptance flag change request to request to set "acceptance" for a pharmacy acceptance flag is received, "acceptance" is set for "pharmacy acceptance flag". If a pharmacy acceptance flag change request to request to set "no acceptance" for a pharmacy acceptance flag is received, "no acceptance" is set for "pharmacy acceptance flag".

In Step S107, the update section 103 updates the pharmacy dispensing status. That is, if "acceptance" or "no acceptance" is set for "pharmacy acceptance flag", the in-pharmacy device 62 sends a pharmacy dispensing status change request to request to set "dispensed" for "pharmacy dispensing status" or a pharmacy dispensing status change request to request to set "non-dispensed" for "pharmacy dispensing status" to the data server 91.

If the communication section 102 receives the pharmacy dispensing status change request sent from the in-pharmacy device 62, the update section 103 identifies a prescription history index, which includes the prescription ID in the pharmacy dispensing status change request, from the prescription history index table 281 of Fig. 13. Then, the update section 103 sets "dispensed" or "non-dispensed" for "pharmacy dispensing status" of the identified prescription history index in response to the pharmacy dispensing status change request.

In Step S108, the data server 91 determines if a request to write an electric medication notebook is made or not. For example, if the in-pharmacy device 62 sends an electric medication notebook writing request to the data server 91, it is determined that a request to write an electric medication notebook is made.

If it is determined that a request to write an electric medication notebook is not made in Step S108, the process returns to Step S101, and the above-mentioned process is repeated.

To the contrary, if it is determined that a request to write an electric medication notebook is made in Step S108, the communication section 102 receives the electric medication notebook writing request from the in-pharmacy device 62, and the process proceeds to Step S109.

In Step S109, the update section 103 writes an electric medication notebook in response to the electric medication notebook writing request. That is, the update section 103 adds a new dispensing history index to the dispensing history index table 301 of Fig. 14, and stores each piece of information in the dispensing history index. For example, the pharmacy name, pharmacist name, and pharmacy contact information, which are in the electric medication notebook writing request, are stored in "pharmacy name", "pharmacist name", and "pharmacy contact information" of the dispensing history index. Further, the update section 103 updates the dispensing history information table 302 of Fig. 14 in response to the electric medication notebook writing request.

An electric medication notebook is written, then the process returns to Step S101, and the above-mentioned process is repeated.

As described above, the data server 91 sends the dispensing reservation information in response to a request from the in-pharmacy device 62, and updates the pharmacy acceptance flag and the pharmacy dispensing status. As described above, the data server 91 supplies information, which is written by the in-hospital device 32, and the like, to the in-pharmacy device 62 as necessary. As a result, a pharmacist is capable of starting to dispense a drag before a patient visits a pharmacy, and convenience for a patient may be improved.

### [About reference to electric medication notebook at pharmacy]

Further, a patient visits a pharmacy, in which the in-pharmacy system 12 is installed, and is capable of displaying an electric medication notebook and the like at the pharmacy.

In this case, a patient passes the IC card 14 that he has over the identification information reader 61. Then, the identification information reader 61 retrieves identification information from the IC card 14 via wireless communication, and supplies the identification information to the in-pharmacy device 62.

The electric medication notebook reference section 71 of the in-pharmacy device 62 controls the communication section 75 to thereby send the identification information, which is supplied from the identification information reader 61, to the data server 91, and requests to send information in the patient basic information database 221 to the pharmacist dispensing information database 223.

The data server 91 receives the identification information sent from the in-pharmacy device 62, then retrieves information requested by the in-pharmacy device 62, and sends information, which is obtained as the result, to the in-pharmacy device 62.

That is, with reference to the identification information/patient correspondence database 211, the data server 91 retrieves database name for each patient, which is identified by the received identification information. Then, the data server 91 extracts information, which is in the patient basic information database 221 to the pharmacist dispensing information database 223, from the patient database 212 of the retrieved database name for each patient, and sends the information to the in-pharmacy device 62.

If the communication section 75 receives information, which is in the patient basic information database 221 to the pharmacist dispensing information database 223 sent from the data server 91, the electric medication notebook reference section 71 processes the information as necessary, and causes the display section 76 to display necessary information. As a result, a patient or a pharmacist is capable of confirming fundamental information on the patient himself, information on prescriptions of the past examination, information on a prescribed drug, information on dispensing history of the prescribed drug, and the like.

Note that information, which is in the patient basic information database 221 to the pharmacist dispensing information database 223, may not be displayed on the in-pharmacy device 62 but may be displayed on the in-pharmacy receipt computer 63.

Further, in a case where a patient receives a drug, of which dispensing is reserved beforehand, at a pharmacy, for example, a pharmacist may store a prescription ID of dispensing reservation information obtained by the in-pharmacy device 62 in relation with the dispensed drug, when the pharmacist dispenses the prescribed drug.

Then, a patient refers to an electric medication notebook by using the IC card 14 when the patient visits a pharmacy, to thereby cause the in-pharmacy device 62 to display a prescription ID. The pharmacist is capable of giving the drug, which is in relation with the displayed prescription ID, to the patient. In this case, it may be confirmed if a patient is the identical person, as necessary.

### [About return-visit reservation]

Meanwhile, the information processing system of Fig. 1 is capable of reserving the next hospital visit (return-visit) after examination of a patient at a hospital, or at accounting after examination. Hereinafter, processes of the respective apparatuses executed in this case will be described.

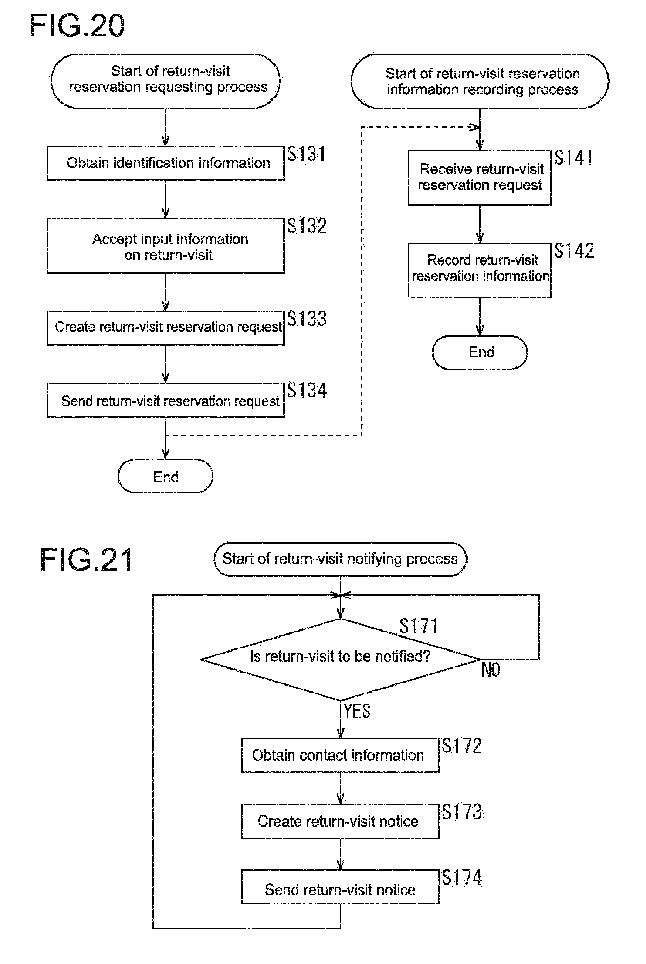

First, with reference to the flowchart of Fig. 20, return-visit reservation requesting process and return-visit reservation information recording process, which are executed by the in-hospital device 32 and the data server 91, will be described.

In Step S131, the return-visit reservation section 44 obtains patient's identification information from the identification information reader 31. That is, in a case of reserving return-visit, a patient passes the IC card 14 that he owns over the identification information reader 31. Then, the identification information reader 31 retrieves identification information from the IC card 14, and supplies the identification information to the in-hospital device 32.

In Step S132, the return-visit reservation section 44 accepts input information on return-visit of a patient. For example, a doctor or the like decides the next hospital-visit appointment day and the like in consultation with a patient, and then operates the in-hospital device 32 to thereby input the next hospital-visit appointment day, a medical institution name (hospital name), a message, a URL of a hospital visit reservation system, and the like. For example, the message is a message from the hospital side such as a doctor to a patient, e.g., "bring insurance card next month" or "bring urine sample".

In Step S133, the return-visit reservation section 44 creates a return-visit reservation request based on input information. The return-visit reservation request includes, for example, the patient's identification information, the next hospital-visit appointment day input by a doctor or the like, the medical institution name, the message, and the hospital visit reservation system URL.

In Step S134, the communication section 45 sends the created return-visit reservation request to the data server 91, and the return-visit reservation requesting process is finished.

In this manner, the in-hospital device 32 sends the created return-visit reservation request to the data server 91, and then the data server 91 starts return-visit reservation information recording process.

In Step S141, the communication section 102 of the data server 91 receives the return-visit reservation request sent from the in-hospital device 32.

In Step S142, the update section 103 records new return-visit reservation information in the return-visit reservation information database 224 of the prescription/dispensing database 101 based on the received return-visit reservation request.

Specifically, the update section 103 identifies the database name for each patient, which is of a patient who reserves return-visit, from the identification information/patient correspondence database 211 based on the identification information in the return-visit reservation request. Then, the update section 103 updates the return-visit reservation information database 224, which constitutes the patient database 212 of the identified name.

For example, the update section 103 adds new return-visit reservation information to the return-visit reservation information database 224 of Fig. 15, and stores a new number in "serial number" of the return-visit reservation information. Further, the update section 103 stores the next hospital-visit appointment day, medical institution name, message, and hospital visit reservation system URL, which are in the return-visit reservation request, in "next hospital-visit appointment day", "medical institution name", "message", and "hospital visit reservation system URL" in the new return-visit reservation information.

In this manner, the return-visit reservation information database 224 is updated, and then the return-visit reservation information recording process is finished.

The return-visit reservation information is recorded in the return-visit reservation information database 224 of the data server 91, and then the information notifying server 92, which monitors the return-visit reservation information database 224, informs (notifies) a patient of return-visit at appropriate timing. For example, return-visit is informed once or several times on a few days before the return-visit appointment day, i.e., the next hospital-visit appointment day, or on the return-visit appointment day.

Hereinafter, with reference to the flowchart of Fig. 21, return-visit notifying process, which is executed by the information notifying server 92 in such a case, will be described.

In Step S171, the monitoring section 104 of the information notifying server 92 refers to the return-visit reservation information database 224 in the patient database 212 of the data server 91, and determines if return-visit is to be notified or not.

That is, the monitoring section 104 obtains the return-visit reservation information database 224 from the data server 91. Then, for example, if return-visit is to be notified the day the before return-visit day, the monitoring section 104 determines that return-visit is to be notified if return-visit reservation information in the return-visit reservation information database 224 includes return-visit reservation information, for which "tomorrow" is set for "next hospital-visit appointment day".

As described above, if "next hospital-visit appointment day" in the return-visit reservation information satisfies a specific condition, it is determined that return-visit is to be notified.

If it is determined that return-visit is not to be notified in Step S171, the process returns to Step S171, and the above-mentioned process is repeated.

Meanwhile, if it is determined that return-visit is to be notified in Step S171, the return-visit notifying section 106 obtains return-visit reservation information, for which "tomorrow" is set for "next hospital-visit appointment day", and the process proceeds to Step S172.

In Step S172, the return-visit notifying section 106 obtains contact information of a patient, who is notified of return-visit. Specifically, the return-visit notifying section 106 refers to the patient database 212 in which return-visit reservation information including "tomorrow", which is set for "next hospital-visit appointment day", is recorded, and obtains a telephone number or an email address of the patient from the patient basic information table 251 of the patient database 212.

In Step S173, the return-visit notifying section 106 creates return-visit notice based on the obtained return-visit reservation information and the patient's contact information.

For example, if return-visit notice is sent by email, the return-visit notifying section 106 creates, as return-visit notice, email, which includes "next hospital-visit appointment day", "medical institution name", "message", and "hospital visit reservation system URL" in the return-visit reservation information, and of which destination is the obtained patient's email address.

Further, for example, if return-visit notice is sent as a voice message by telephone, the return-visit notifying section 106 creates, as return-visit notice, a voice message, which announces "next hospital-visit appointment day", "medical institution name", and "message" in the return-visit reservation information to a patient.

In Step S174, the communication section 105 sends the created return-visit notice, then the process returns to Step S171, and the above-mentioned process is repeated.

For example, if the return-visit notice is email, the communication section 105 sends email to the mobile terminal device 15 of a patient via the communication system 16. Further, if the return-visit notice is a voice message, the communication section 105 sends (transmits), as return-visit notice, a voice message to the mobile terminal device 15, which is identified by the patient's telephone number obtained as patient's contact information.

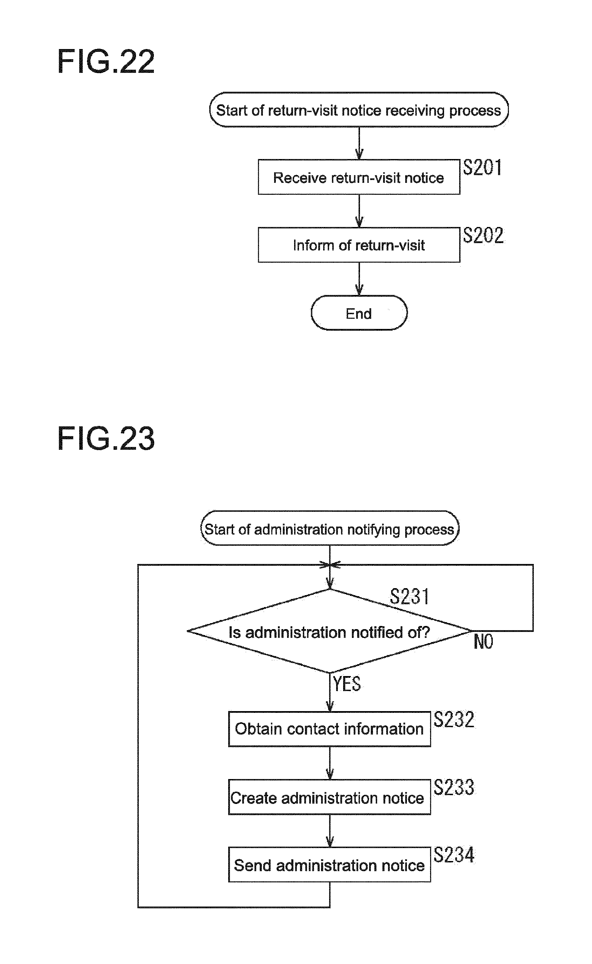

In this manner, the return-visit notice is sent to the mobile terminal device 15 of a patient, and then the mobile terminal device 15 executes return-visit notice receiving process to thereby receive the sent return-visit notice, and informs a patient. Hereinafter, with reference to the flowchart of Fig. 22, return-visit notice receiving process executed by the mobile terminal device 15 will be described.

In Step S201, the communication section 121 receives return-visit notice, which is sent from the information notifying server 92, via the communication system 16 and the like, and supplies the return-visit notice to the controller section 125.

In Step S202, the controller section 125 causes the display section 126 to display the supplied return-visit notice or causes the speaker 123 to output the supplied return-visit notice to thereby inform a patient of return-visit, and the return-visit notice receiving process is finished.

For example, if the return-visit notice is email, the communication section 121 receives email sent from the information notifying server 92, and supplies the email to the controller section 125. Then, the controller section 125 supplies the supplied email to the display section 126, and causes the display section 126 to display the supplied email. Because of this, a patient confirms what is written in the email displayed on the display section 126, to thereby capable of knowing that the return-visit appointment day is tomorrow.

Note that a patient may operate the mobile terminal device 15, and may select a hospital visit reservation system URL (Uniform Resource Locator) displayed on the email, to thereby access a not-shown hospital visit reservation system via the communication system 16 and change the return-visit appointment day.

Further, for example, if return-visit notice is a voice message, the communication section 121 receives a voice message sent from the information notifying server 92, and supplies the voice message to the controller section 125. The controller section 125 supplies predetermined sound data to the speaker 123, and causes the speaker 123 to output a ringtone. When a patient operates the mobile terminal device 15 and telephone call is accepted, the controller section 125 supplies a voice message as return-visit notice to the speaker 123, and causes the speaker 123 to output the voice message. Note that, at this time, if a patient operates the mobile terminal device 15, call may be transferred to an outpatient reservation center, a hospital visit reservation system, or the like, and the patient may be capable of changing the return-visit appointment day.

As described above, the in-hospital device 32 sends a return-visit reservation request to the data server 91, and causes the data server 91 to record return-visit reservation information, which is created based on the return-visit reservation request. The information notifying server 92 monitors the return-visit reservation information in the data server 91. Then, at the timing when return-visit is to be notified, the information notifying server 92 creates return-visit notice based on the return-visit reservation information, and sends the return-visit notice to the mobile terminal device 15.

In this manner, return-visit notice is sent at appropriate timing to thereby inform a patient of return-visit day and the like, whereby it is possible to increase possibility of maintenance of therapy to a patient at appropriate return-visit timing, and it is possible to gain treatment effects of medical practice at maximum. Further, if a patient is informed of the return-visit day at appropriate timing, the patient may not forget the return-visit day and the like, and it is possible to improve convenience for a patient.

Further, the hospital side is capable of transmitting a return-visit day and a message to a patient reliably, and it is possible to increase return-visit rate of a patient, who has a chronic disorder, and the like, specifically.

The information processing system notifies a patient of return-visit reservation based on the return-visit reservation information database 224, whereby it is possible to notify a patient, to whom a prescription is not issued, of return-visit reservation.

Note that the information notifying server 92 may extract the number of days of prescription, frequency of use, quantity per dose, prescription information writing date/time, and the like of a prescribed drug from the doctor prescription information database 222 and the dispensing history information table 302 in the patient database 212, and may urge a patient to reserve return-visit by using the information.

In this case, for example, the return-visit notifying section 106 sends email, which includes a hospital visit reservation system URL and urges to reserve next hospital visit, to the mobile terminal device 15 of a patient. A patient confirms what is written in the email, operates the mobile terminal device 15 to thereby access the hospital visit reservation system, and is capable of reserving return-visit. Here, the timing to send email, which urges to reserve next hospital visit, is obtained based on the day, on which a patient finishes dosing prescribed drugs, which is calculated based on the number of days of prescription of the prescribed drugs and the like, for example.

Further, the mobile terminal device 15 may obtain return-visit reservation information from the data server 91, and may inform a patient of return-visit once or several times at appropriate timing.

### [About administration notice]

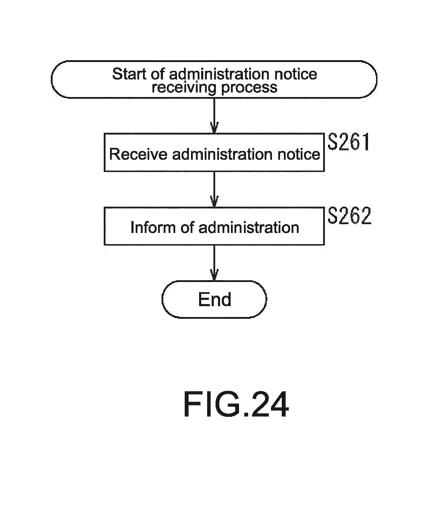

Further, the information notifying server 92 monitors the patient database 212 of the data server 91, and notifies a patient of administration of a drug when a drug prescribed to a patient is to be administered. Hereinafter, with reference to Fig. 23 and Fig. 24, process executed by the information notifying server 92 and the mobile terminal device 15 in this case will be described.

Fig. 23 is a flowchart for explaining administration notifying process executed by the information notifying server 92.

In Step S231, the monitoring section 104 of the information notifying server 92 refers to the pharmacist dispensing information database 223 and the patient basic information table 251, which are in the patient database 212 of the data server 91, and determine if administration is notified of.

For example, the monitoring section 104 obtains the dispensing history index table 301 and the dispensing history information table 302 of Fig. 14, and the patient basic information table 251 of Fig. 12 from the prescription/dispensing database 101.

Then, the monitoring section 104 identifies a drug, which a patient is administering, based on "dispensing information writing date/time" in the dispensing history index of the dispensing history index table 301, and based on "number of days of dispensing" in the dispensing history information of the dispensing history information table 302. Specifically, if "dispensing information writing date/time" is before a day, which is before the present date/time by "number of days of dispensing", then it means that the drug administering period has passed.

If a drug being administered is identified, the monitoring section 104 identifies if it is the time to administer a drug by a patient based on "frequency of use" of dispensing history information of the drug, and based on "breakfast time", "lunchtime", "supper time", and "time of sleep" of the patient basic information table 251.

Specifically, for example, in a case where "frequency of use" of dispensing history information is "before going to bed", if the present time is before "time of sleep" of the patient basic information table 251 by a preset period of time, then it is determined that it is time to administer a drug by a patient.

In this manner, if dispensing history information and information on administration timing of the patient basic information table 251 satisfy specific conditions, and if it is time to administer a drug by a patient, then it is determined in Step S231 that administration of the drug is to be notified.

If it is determined that administration is not to be notified in Step S231, the process returns to Step S231, and the above-mentioned process is repeated.

Meanwhile, if it is determined that administration is to be notified in Step S231, the administration notifying section 107 obtains dispensing history information of a drug, of which administration is to be notified, from the dispensing history information table 302 of the data server 91, and the process proceeds to Step S232.

In Step S232, the administration notifying section 107 obtains patient's contact information, which is the administration notifying target. Specifically, the administration notifying section 107 refers to the patient database 212, in which the obtained dispensing history information is recorded, and obtains a patient's telephone number or email address from the patient basic information table 251 of the patient database 212.

In Step S233, the administration notifying section 107 creates administration notice based on the obtained dispensing history information and patient's contact information.

For example, if administration notice is sent by email, the administration notifying section 107 creates email, of which destination is the obtained patient's email address, as administration notice. The email includes a message to urge to administer a drug, which is identified by "dispensed drug identification ID" of dispensing history information, by the amount of "quantity per dose" of dispensing history information, and includes a real image of the drug to be administered.

Note that the drug name corresponding to the dispensed drug identification ID and the image of the drug may be previously recorded in the information notifying server 92, or may be obtained from the data server 91 by the information notifying server 92.

Further, for example, if administration notice is sent as a voice message by telephone, the administration notifying section 107 creates, as administration notice, a voice message, which urges to administer a drug identified by "dispensed drug identification ID" of dispensing history information by the amount of "quantity per dose" of dispensing history information.

In Step S234, the communication section 105 sends the created administration notice, the process returns to Step S231, and the above-mentioned process is repeated.

For example, if administration notice is email, the information notifying server 92 sends email to the mobile terminal device 15 of a patient via the communication system 16. Further, if administration notice is a voice message, the information notifying server 92 sends (transmits) a voice message as administration notice to the mobile terminal device 15, which is identified by the patient's telephone number obtained as patient's contact information.

In this manner, the administration notice is sent to the mobile terminal device 15 of a patient, and then the mobile terminal device 15 executes administration notice receiving process to thereby receive the sent administration notice, and informs a patient of administration. Hereinafter, with reference to the flowchart of Fig. 24, the administration notice receiving process executed by the mobile terminal device 15 will be described.

In Step S261, the communication section 121 receives administration notice, which is sent from the information notifying server 92, via the communication system 16 and the like, and supplies the administration notice to the controller section 125.

In Step S262, the controller section 125 causes the display section 126 to display the supplied administration notice, or causes the speaker 123 to output the supplied administration notice to thereby inform of administration, and the administration notice receiving process is finished.

For example, if administration notice is email, the communication section 121 receives email sent from the information notifying server 92, and supplies the email to the controller section 125. Then, the controller section 125 supplies the supplied email to the display section 126, and causes the display section 126 to display the email. As a result, a patient confirms what is written in the email displayed on the display section 126, to thereby capable of knowing that it is time to administer the drug.

Further, for example, if return-visit notice is a voice message, the communication section 121 receives a voice message sent from the information notifying server 92, and supplies the voice message to the controller section 125. The controller section 125 supplies predetermined sound data to the speaker 123, and causes the speaker 123 to output a ringtone. When a patient operates the mobile terminal device 15 and telephone call is accepted, the controller section 125 supplies a voice message as administration notice to the speaker 123, and causes the speaker 123 to output the voice message.

As described above, the information notifying server 92 monitors the patient database 212 of the data server 91, creates administration notice based on dispensing history information at the timing when administration is to be notified, and sends the administration notice to the mobile terminal device 15.

As described above, administration notice is sent at appropriate timing to thereby inform a patient of administration time, whereby it is possible to send detailed administration urging information to the mobile terminal device 15 of a patient. As a result, a patient is capable of administering a drug properly, and it is possible to improve convenience for a patient. Further, not only the name of a drug to be administered but also the image of the drug is displayed in the administration notice, whereby a patient is capable of administering a drug easily and without making a mistake even if he does not know the name of the drug.

Further, because of administration notice, it is possible to improve patient's drug compliance, and to help to onset of treatment effects intended by a doctor. As a result, it is possible to gain treatment effects of medical practice at maximum.

Note that the mobile terminal device 15 may obtain a dispensing history index, dispensing history information, a patient basic table, and the like from the data server 91, and may inform of administration once or several times at appropriate timing.

The above-mentioned series of processes may be executed by hardware or software. If the series of processes is executed by software, a program configuring the software in a program recording medium is installed in a computer, which is mounted on dedicated hardware, or in, for example, a general-purpose personal computer, in which various programs are installed and which is capable of executing various functions.

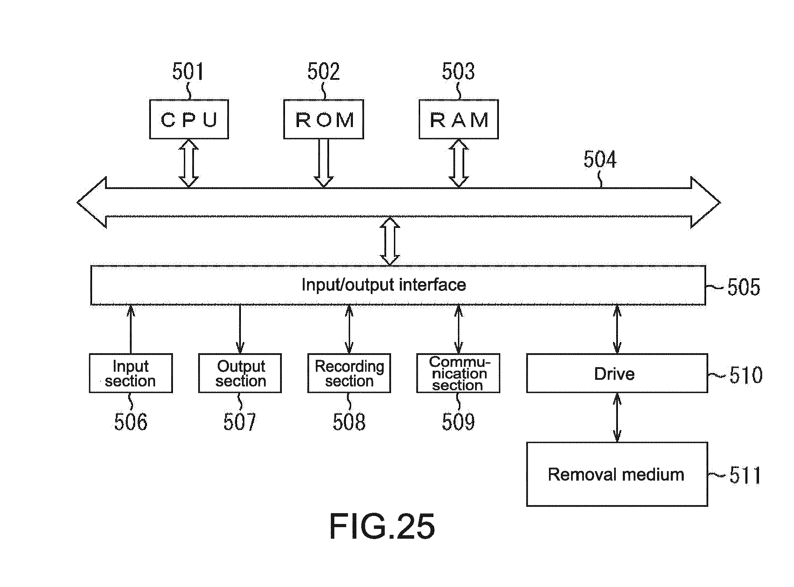

Fig. 25 is a block diagram showing a configuration example of hardware of a computer executing the above-mentioned series of processes by means of a program.

The computer includes a CPU (Central Processing Unit) 501, a ROM (Read Only Memory) 502, and a RAM (Random Access Memory) 503, which are connected each other by a bus 504.

Further, an input/output interface 505 is connected to the bus 504. An input section 506 including a keyboard, a mouse, a microphone, and the like, an output section 507 including a display, a speaker, and the like, a recording section 508 including a hard disk, a nonvolatile memory, and the like, a communication section 509 including a network interface and the like, and a drive 510 for driving a removal medium 511 such as a magnetic disk, an optical disk, a magnet-optical disk, or a semiconductor memory, are connected to the input/output interface 505.

In the computer configured as described above, for example, the CPU 501 loads programs, which are recorded in the recording section 508, in the RAM 503 via the input/output interface 505 and the bus 504, to thereby execute the above-mentioned series of processes.

The programs executed by the computer (CPU 501) is recorded in the removal medium 511, which is a package medium, i.e., a magnetic disk (including flexible disk), an optical disk (CD-ROM (Compact Disc-Read Only Memory), DVD (Digital Versatile Disc), etc.), a magnet-optical disk, a semiconductor memory, or the like, for example. Alternatively, the programs are supplied via a wired or wireless transmission medium such as a local area network, the Internet, or digital satellite broadcasting.

Further, the removal medium 511 is mounted in the drive 510, whereby the programs may be installed in the recording section 508 via the input/output interface 505. Further, the programs may be received by the communication section 509 via a wired or wireless transmission medium, and may be installed in the recording section 508. Alternatively, the programs may be previously installed in the ROM 502 or the recording section 508.

Note that the programs may be executed by the computer sequentially in the order described in this specification, in parallel, or at necessary timing, i.e., when the programs are called.

Note that the embodiment of the present invention is not limited to the above-mentioned embodiment, but various changes may be made without departing from the scope of the invention.

### Description of Numerals

- 32: in-hospital device
- 33: in-hospital receipt computer
- 41: electric medication notebook reference section
- 42: electric prescription writing section
- 43: dispensing reservation section
- 44: return-visit reservation section
- 45: communication section
- 46: display section
- 47: in-hospital database
- 62: in-pharmacy apparatus
- 71: electric medication notebook reference section
- 72: electric prescription capturing section
- 73: electric medication notebook writing section
- 74: dispensing reservation section
- 75: communication section
- 76: display section
- 77: in-pharmacy database
- 91: data server
- 92: information notifying server
- 101: prescription/dispensing database
- 102: communication section
- 103: update section
- 104: monitoring section
- 105: communication section
- 106: return-visit notifying section
- 107: administration notifying section

## Claims

1. An information processing apparatus, comprising:
a creating means for creating a dispensing reservation request, the dispensing reservation request requesting to reserve dispensing of a drug prescribed based on a prescription issued to a patient, the dispensing reservation request including identification information identifying the patient, prescription information on the prescription, and drug identification information identifying the drug of which reservation is requested; and
a sending means for sending the dispensing reservation request.

2. The information processing apparatus according to claim 1, further comprising
an obtaining means for causing a server managing the prescription information to send the identification information, to thereby obtain the prescription information of the patient from the server.

3. The information processing apparatus according to claim 2, wherein
the drug identification information is information identifying the type of drug, or information showing acceptance/no acceptance of change to generics.

4. The information processing apparatus according to claim 2, wherein
the obtaining means further obtains dispensing history information on the drug prescribed to the patient, from the server,
the information processing apparatus further comprises a display means for displaying a list of pharmacies to which dispensing of drugs was requested by the patient in the past, the dispensing history information including the list, and
the dispensing reservation request further includes pharmacy identification information identifying a pharmacy to which dispensing reservation of the drug is requested, the pharmacy being selected from the list of the pharmacies.

5. An information processing method executed by an information processing apparatus, including
a creating means for creating a dispensing reservation request, the dispensing reservation request requesting to reserve dispensing of a drug prescribed based on a prescription issued to a patient, the dispensing reservation request including identification information identifying the patient, prescription information on the prescription, and drug identification information identifying the drug of which reservation is requested, and
a sending means for sending the dispensing reservation request, the information processing method comprising the steps of:
creating, by the creating means, the dispensing reservation request; and
sending, by the sending means, the dispensing reservation request.

6. A program, causing a computer to execute a process comprising the steps of:
creating a dispensing reservation request, the dispensing reservation request requesting to reserve dispensing of a drug prescribed based on a prescription issued to a patient, the dispensing reservation request including identification information identifying the patient, prescription information on the prescription, and drug identification information identifying the drug of which reservation is requested; and
sending the dispensing reservation request.

7. An information processing apparatus, comprising:
a recording means for recording prescription information on a prescription issued to a patient and dispensing history information on a drug prescribed to the patient, in relation with identification information identifying the patient;
a sending means for sending, in a case where a request to send the prescription information and the dispensing history information including the identification information is received, the prescription information and the dispensing history information recorded in relation with the identification information, to an apparatus that sent the request, in response to the request;
a receiving means for receiving a dispensing reservation request sent from the apparatus, the dispensing reservation request requesting to dispensing reservation of a drug prescribed based on the prescription, the dispensing reservation request including pharmacy identification information identifying a pharmacy to which dispensing reservation of a drug is requested, the pharmacy being selected from pharmacies to which dispensing of drugs was requested by the patient in the past, the pharmacies being in at least the identification information, the prescription information, and the dispensing history information; and
a creating means for creating, in response to the dispensing reservation request, prescription identification information identifying the prescription, drug identification information identifying a drug of which dispensing reservation is requested, and dispensing reservation information including the pharmacy identification information, and for causing the recording means to record the dispensing reservation information in relation with the identification information, wherein
the sending means sends the dispensing reservation information to an apparatus at a pharmacy identified by the pharmacy identification information, and sends the prescription information of a prescription identified by the prescription identification information in the dispensing reservation information, to the apparatus at the pharmacy, in response to a request from the apparatus at the pharmacy.

8. The information processing apparatus according to claim 7, wherein
the drug identification information is information identifying the type of drug, or information showing acceptance/no acceptance of change to generics.

9. An information processing method executed by an information processing apparatus, including
a recording means for recording prescription information on a prescription issued to a patient and dispensing history information on a drug prescribed to the patient, in relation with identification information identifying the patient,
a sending means for sending, in a case where a request to send the prescription information and the dispensing history information including the identification information is received, the prescription information and the dispensing history information recorded in relation with the identification information, to an apparatus that sent the request, in response to the request,
a receiving means for receiving a dispensing reservation request sent from the apparatus, the dispensing reservation request requesting to dispensing reservation of a drug prescribed based on the prescription, the dispensing reservation request including pharmacy identification information identifying a pharmacy to which dispensing reservation of a drug is requested, the pharmacy being selected from pharmacies to which dispensing of drugs was requested by the patient in the past, the pharmacies being in at least the identification information, the prescription information, and the dispensing history information, and
a creating means for creating, in response to the dispensing reservation request, prescription identification information identifying the prescription, drug identification information identifying a drug of which dispensing reservation is requested, and dispensing reservation information including the pharmacy identification information, and for causing the recording means to record the dispensing reservation information in relation with the identification information,
the sending means sending the dispensing reservation information to an apparatus at a pharmacy identified by the pharmacy identification information, and sending the prescription information of a prescription identified by the prescription identification information in the dispensing reservation information, to the apparatus at the pharmacy, in response to a request from the apparatus at the pharmacy,
the information processing method comprising the steps of:
recording, by the recording means, the prescription information and the dispensing history information in relation with the identification information;
sending, by the sending means, the prescription information and the dispensing history information in response to the request;
receiving, by the receiving means, the dispensing reservation request;
creating, by the creating means, the dispensing reservation information;
sending, by the sending means, the dispensing reservation information to an apparatus at a pharmacy identified by the pharmacy identification information, and sending the prescription information of a prescription identified by the prescription identification information in the dispensing reservation information, to the apparatus at the pharmacy, in response to a request from the apparatus at the pharmacy.

10. A program, causing a computer to execute a process comprising the steps of:
recording prescription information on a prescription issued to a patient and dispensing history information on a drug prescribed to the patient, in relation with identification information identifying the patient;
sending, in a case where a request to send the prescription information and the dispensing history information including the identification information is received, the prescription information and the dispensing history information recorded in relation with the identification information, to an apparatus that sent the request, in response to the request;
receiving a dispensing reservation request sent from the apparatus, the dispensing reservation request requesting to dispensing reservation of a drug prescribed based on the prescription, the dispensing reservation request including pharmacy identification information identifying a pharmacy to which dispensing reservation of a drug is requested, the pharmacy being selected from pharmacies to which dispensing of drugs was requested by the patient in the past, the pharmacies being in at least the identification information, the prescription information, and the dispensing history information;
creating, in response to the dispensing reservation request, prescription identification information identifying the prescription, drug identification information identifying a drug of which dispensing reservation is requested, and dispensing reservation information including the pharmacy identification information, and causing the recording means to record the dispensing reservation information in relation with the identification information; and
sending the dispensing reservation information to an apparatus at a pharmacy identified by the pharmacy identification information, and sending the prescription information of a prescription identified by the prescription identification information in the dispensing reservation information, to the apparatus at the pharmacy, in response to a request from the apparatus at the pharmacy.

11. An information processing apparatus, comprising:
a receiving means for receiving, from a server managing prescription information on a prescription issued to a patient in relation with identification information identifying the patient, dispensing reservation information including prescription identification information identifying the prescription in the prescription information, and including drug identification information identifying a drug of which dispensing reservation is requested;
an obtaining means for obtaining, from the server, the prescription information on the prescription identified by the prescription identification information, based on the prescription identification information in the dispensing reservation information; and
a sending means for sending information indicating that the drug dispensing reservation is accepted, to the server.

12. The information processing apparatus according to claim 11, wherein
the drug identification information is information identifying the type of drug, or information showing acceptance/no acceptance of change to generics.

13. The information processing apparatus according to claim 11, further comprising
a recording means for recording stock information showing stock status of drugs at the predetermined pharmacy, wherein
the sending means sends, in a case where a stock amount of the drug indicated by the stock information is larger than the amount of the drug prescribed based on the prescription corresponding to the prescription information, information that the drug dispensing reservation is accepted.

14. An information processing method executed by an information processing apparatus, including
a receiving means for receiving, from a server managing prescription information on a prescription issued to a patient in relation with identification information identifying the patient, dispensing reservation information including prescription identification information identifying the prescription in the prescription information, and including drug identification information identifying a drug of which dispensing reservation is requested,
an obtaining means for obtaining, from the server, the prescription information on the prescription identified by the prescription identification information, based on the prescription identification information in the dispensing reservation information, and
a sending means for sending information indicating that the drug dispensing reservation is accepted, to the server,
the information processing method comprising the steps of:
receiving, by the receiving means, the dispensing reservation information;
obtaining, by the obtaining means, the prescription information; and
sending, by the sending means, information indicating that the drug dispensing reservation is accepted, to the server.

15. A program, causing a computer to execute a process comprising the steps of:
receiving, from a server managing prescription information on a prescription issued to a patient in relation with identification information identifying the patient, dispensing reservation information including prescription identification information identifying the prescription in the prescription information, and including drug identification information identifying a drug of which dispensing reservation is requested;
obtaining, from the server, the prescription information on the prescription identified by the prescription identification information, based on the prescription identification information in the dispensing reservation information; and
sending information indicating that the drug dispensing reservation is accepted, to the server.

16. An information processing apparatus, comprising:
an obtaining means for obtaining, from a recording means recording examination reservation information on examination reservation including an examination appointment day of a patient at a medical institution in relation with identification information identifying the patient, the examination reservation information;
a creating means for creating an examination reservation notice notifying the patient of the examination appointment day based on the examination reservation information, in a case where the examination appointment day in the examination reservation information satisfies a predetermined condition; and
a sending means for sending the examination reservation notice to the patient's contact information identified by the identification information in relation with the examination reservation information, as a destination.

17. The information processing apparatus according to claim 16, wherein
the creating means creates email or a voice message, as the examination reservation notice.

18. The information processing apparatus according to claim 16,
wherein
the examination reservation information includes a message from the medical institution to the patient, and the creating means creates the examination reservation notice including the message.

19. An information processing method executed by an information processing apparatus, including
an obtaining means for obtaining, from a recording means recording examination reservation information on examination reservation including an examination appointment day of a patient at a medical institution in relation with identification information identifying the patient, the examination reservation information,
a creating means for creating an examination reservation notice notifying the patient of the examination appointment day based on the examination reservation information, in a case where the examination appointment day in the examination reservation information satisfies a predetermined condition, and
a sending means for sending the examination reservation notice to the patient's contact information identified by the identification information in relation with the examination reservation information, as a destination,
the information processing method comprising the steps of:
obtaining, by the obtaining means, the examination reservation information;
creating, by the creating means, the examination reservation notice; and
sending, by the sending means, the examination reservation notice.

20. A program, causing a computer to execute a process comprising the steps of:
obtaining, from a recording means recording examination reservation information on examination reservation including an examination appointment day of a patient at a medical institution in relation with identification information identifying the patient, the examination reservation information;
creating an examination reservation notice notifying the patient of the examination appointment day based on the examination reservation information, in a case where the examination appointment day in the examination reservation information satisfies a predetermined condition; and
sending the examination reservation notice to the patient's contact information identified by the identification information in relation with the examination reservation information, as a destination.

21. An information processing apparatus, comprising:
a receiving means for receiving, from a server managing examination reservation information on examination reservation including an examination appointment day of a patient at a medical institution in relation with identification information identifying the patient, examination reservation notice notifying the patient of the examination appointment day, the examination reservation notice being sent in a case where the examination appointment day in the examination reservation information satisfies a predetermined condition; and
an informing means for informing the patient of the examination appointment day based on the received examination reservation notice.

22. The information processing apparatus according to claim 21, wherein
the examination reservation notice is email, and the informing means displays the email to thereby inform the patient of the examination appointment day.

23. The information processing apparatus according to claim 21, wherein
the examination reservation notice is a voice message, and the informing means outputs the voice message to thereby inform the patient of the examination appointment day.

24. An information processing method executed by an information processing apparatus including
a receiving means for receiving, from a server managing examination reservation information on examination reservation including an examination appointment day of a patient at a medical institution in relation with identification information identifying the patient, examination reservation notice notifying the patient of the examination appointment day, the examination reservation notice being sent in a case where the examination appointment day in the examination reservation information satisfies a predetermined condition, and
an informing means for informing the patient of the examination appointment day based on the received examination reservation notice,
the information processing method comprising the steps of:
receiving, by the receiving means, the examination reservation notice; and
informing, by the informing means, the patient of the examination appointment day.

25. A program, causing a computer to execute a process comprising the steps of:
receiving, from a server managing examination reservation information on examination reservation including an examination appointment day of a patient at a medical institution in relation with identification information identifying the patient, examination reservation notice notifying the patient of the examination appointment day, the examination reservation notice being sent in a case where the examination appointment day in the examination reservation information satisfies a predetermined condition; and
informing the patient of the examination appointment day based on the received examination reservation notice.

26. An information processing apparatus, comprising:
an obtaining means for obtaining, from a recording means recording dispensing history information on a drug including information on administration timing of the drug prescribed to a patient in relation with identification information identifying the patient, the dispensing history information;
a creating means for creating an administration notice urging the patient to administer the drug based on the dispensing history information, in a case where information showing the administration timing in the dispensing history information satisfies a predetermined condition; and
a sending means for sending the administration notice to the patient's contact information identified by the identification information in relation with the dispensing history information, as a destination.

27. The information processing apparatus according to claim 26, wherein
the creating means creates email including the amount of the drug to be administered and an image of the drug, as the administration notice.

28. The information processing apparatus according to claim 26, wherein
the creating means creates a voice message urging the patient to administer the drug by a predetermined amount, as the administration notice.

29. An information processing method executed by an information processing apparatus including
an obtaining means for obtaining, from a recording means recording dispensing history information on a drug including information on administration timing of the drug prescribed to a patient in relation with identification information identifying the patient, the dispensing history information,
a creating means for creating an administration notice urging the patient to administer the drug based on the dispensing history information, in a case where information showing the administration timing in the dispensing history information satisfies a predetermined condition, and
a sending means for sending the administration notice to the patient's contact information identified by the identification information in relation with the dispensing history information, as a destination,
the information processing method comprising the steps of:
obtaining, by the obtaining means, the dispensing history information;
creating, by the creating means, the administration notice; and
sending, by the sending means, the administration notice.

30. A program, causing a computer to execute a process comprising the steps of:
obtaining, from a recording means recording dispensing history information on a drug including information on administration timing of the drug prescribed to a patient in relation with identification information identifying the patient, the dispensing history information;
creating an administration notice urging the patient to administer the drug based on the dispensing history information, in a case where information showing the administration timing in the dispensing history information satisfies a predetermined condition; and
sending the administration notice to the patient's contact information identified by the identification information in relation with the dispensing history information, as a destination.

31. An information processing apparatus, comprising:
a receiving means for receiving, from a server managing dispensing history information on the drug including information showing administration timing of a drug prescribed to a patient in relation with identification information identifying the patient, an administration notice urging the patient to administer the drug, the administration notice being sent in a case where information indicating the administration timing in the dispensing history information satisfies a predetermined condition; and
an informing means for informing the patient of administration of the drug based on the received administration notice.

32. The information processing apparatus according to claim 31, wherein
the administration notice is email including the amount of the drug to be administered and an image of the drug, and the informing means displays the email to thereby inform the patient of administration of the drug.

33. The information processing apparatus according to claim 31, wherein
the administration notice is a voice message urging the patient to administer the drug by a predetermined amount, and the informing means outputs the voice message to thereby inform the patient of administration of the drug.

34. An information processing method executed by an information processing apparatus including
a receiving means for receiving, from a server managing dispensing history information on the drug including information showing administration timing of a drug prescribed to a patient in relation with identification information identifying the patient, an administration notice urging the patient to administer the drug, the administration notice being sent in a case where information indicating the administration timing in the dispensing history information satisfies a predetermined condition, and
an informing means for informing the patient of administration of the drug based on the received administration notice,
the information processing method comprising the steps of:
receiving, by the receiving means, the administration notice; and
informing, by the informing means, the patient of administration of the drug.

35. A program, causing a computer to execute a process comprising the steps of:
receiving, from a server managing dispensing history information on the drug including information showing administration timing of a drug prescribed to a patient in relation with identification information identifying the patient, an administration notice urging the patient to administer the drug, the administration notice being sent in a case where information indicating the administration timing in the dispensing history information satisfies a predetermined condition; and
informing the patient of administration of the drug based on the received administration notice.
